(19) Europäisches Patentamt
European Patent Office
Office européen des brevets

(11) Veröffentlichungsnummer: **0 427 031 A2**

# (12) EUROPÄISCHE PATENTANMELDUNG

(21) Anmeldenummer: **90120149.1**

(22) Anmeldetag: **20.10.90**

(51) Int. Cl.5: **C07D 413/04**, C07D 265/36, A01N 43/84

(30) Priorität: **04.11.89 DE 3936826**

(43) Veröffentlichungstag der Anmeldung: **15.05.91 Patentblatt 91/20**

(84) Benannte Vertragsstaaten: **BE CH DE FR GB IT LI NL**

(71) Anmelder: **BAYER AG**

**W-5090 Leverkusen 1 Bayerwerk(DE)**

(72) Erfinder: **Kunisch, Franz, Dr.**
**Zum Hahnenberg 20**
**W-5068 Odenthal(DE)**
Erfinder: **Lürssen, Klaus, Dr.**
**August-Kierspel-Strasse 145**
**W-5060 Bergisch Gladbach 2(DE)**
Erfinder: **Santel, Hans-Joachim, Dr.**
**Grünstrasse 9a**
**W-5090 Leverkusen 1(DE)**
Erfinder: **Schmidt, Robert R., Dr.**
**Im Waldwinkel 110**
**W-5060 Bergisch Gladbach 2(DE)**

## (54) N-Aryl-Stickstoffheterocyclen.

(57) Neue N-Aryl-Stickstoffheterocyclen der Formel (I)

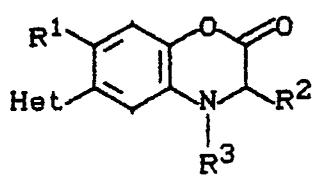

(I)

in welcher
Het für einen Heterocyclus der Formel



Xerox Copy Centre

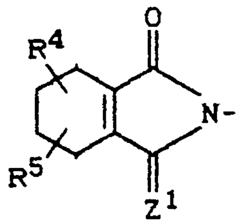 ; 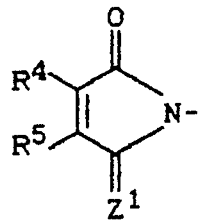 ; 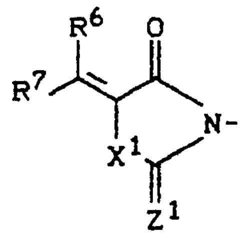 ;

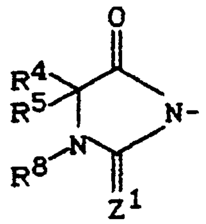 ; 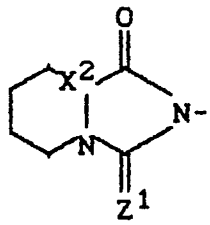 ; 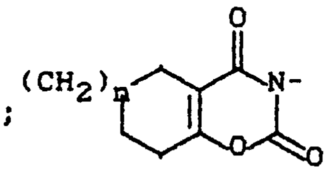 ;

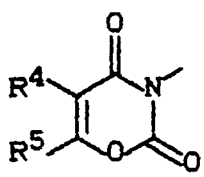 ; 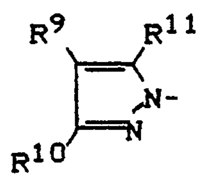 ; 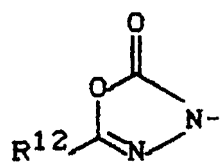 ;

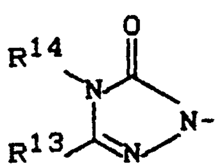 oder 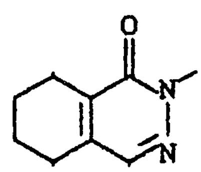

steht,
mehrere Verfahren sowie neue Zwischenprodukte zu ihrer Herstellung und ihre Verwendung als Herbizide.

# N-ARYL-STICKSTOFFHETEROCYCLEN

Die Erfindung betrifft neue N-Aryl-Stickstoffheterocyclen, mehrere Verfahren sowie neue Zwischenprodukte zu ihrer Herstellung und ihre Verwendung als Herbizide.

Es ist bereits bekannt, daß bestimmte N-Aryl-Stickstoffheterocyclen herbizide Eigenschaften besitzen (vgl. z.B. EP-A 61 741, EP-A 83 055 oder EP-A 200 872).

Es wurden neue N-Aryl-Stickstoffheterocyclen der allgemeinen Formel (I),

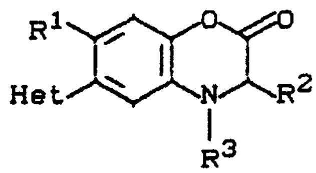

(I)

in welcher

Het für einen Heterocyclus der Formel

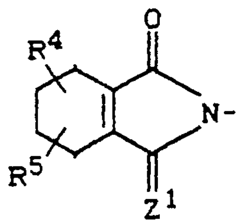 ; 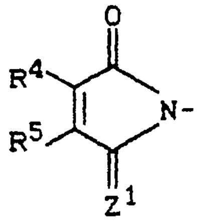 ; 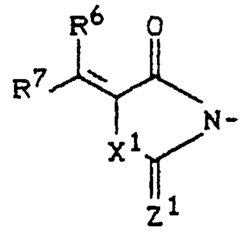 ;

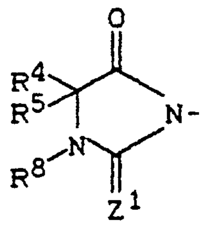 ; 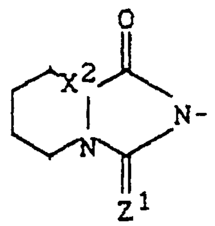 ; 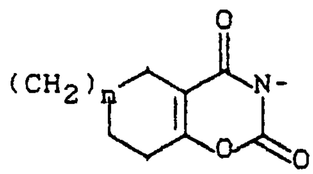 ;

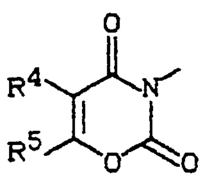 ; 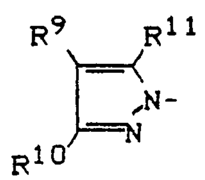 ; 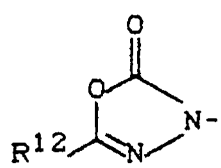 ;

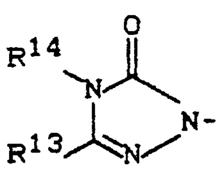 oder 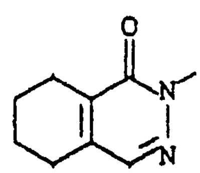 steht,

$R^1$ für Wasserstoff oder Halogen steht,
$R^2$ für Wasserstoff oder Alkyl steht,
$R^3$ für Wasserstoff oder jeweils gegebenenfalls substituiertes Alkyl, Alkenyl, Alkinyl, Cycloalkyl, Cycloalkenyl, Alkylsulfonyl, Arylsulfonyl, Alkylcarbonyl oder Alkoxycarbonyl steht,
$R^4$ und $R^5$ unabhängig voneinander jeweils für Wasserstoff oder Alkyl stehen,
$R^6$ und $R^7$ entweder unabhängig voneinander jeweils für Wasserstoff oder Alkyl stehen oder gemeinsam für einen zweifach verknüpften Alkandiylrest stehen,
$R^8$ für Wasserstoff, Alkyl oder gegebenenfalls substituiertes Aryl steht,
$R^9$ für Wasserstoff, Halogen, Alkyl oder Halogenalkyl steht,
$R^{10}$ für Wasserstoff, Alkyl oder Halogenalkyl steht oder
$R^9$ und $R^{10}$ gemeinsam für zweifach verknüpftes Alkandiyl stehen,
$R^{11}$ für Wasserstoff, Halogen, Alkyl oder Halogenalkyl steht,
$R^{12}$ und $R^{13}$ unabhängig voneinander für Wasserstoff, Alkyl, Alkoxyalkyl, Halogenalkyl, Alkenyl, Halogenalkenyl, Alkinyl, Halogenalkinyl oder für gegebenenfalls substituiertes Cycloalkyl stehen,
$R^{14}$ für Wasserstoff, Alkyl, Halogenalkyl, Alkenyl, Halogenalkenyl, Alkinyl oder Halogenalkinyl steht oder
$R^{13}$ und $R^{14}$ gemeinsam für zweifach verknüpftes Alkandiyl stehen,
$X^1$ für Sauerstoff, eine $-CH_2$-Gruppe oder eine

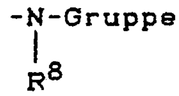

steht,
$X^2$ für Stickstoff oder eine CH-Gruppe steht,
$Z^1$ für Sauerstoff oder Schwefel steht und
n für die Zahl 0 oder 1 steht,
gefunden.

Die Reste ($R^1$, $R^2$, Het usw.) die in den Wirkstoffen der Formel (I) definiert sind, haben in den Zwischen- und Vorprodukten für alle Definitionsbereiche ebenfalls die bei den Verbindungen der Formel (I) angegebenen Bedeutungen. Entsprechendes gilt auch für die Reste, die in Vor- und Zwischenprodukten mehrmals genannt sind.

Weiterhin wurde gefunden, das man die neuen N-Aryl-Stickstoffheterocyclen der Formel (I) nach einem der im folgenden beschriebenen Verfahren erhält:

(a) Man erhält N-Aryl-Sticktoffheterocyclen der Formel (Ia)

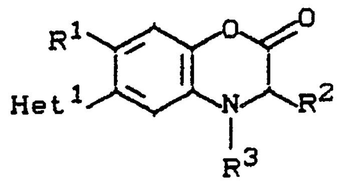

(Ia)

in welcher
$Het^1$ für einen Heterocyclus der Formel

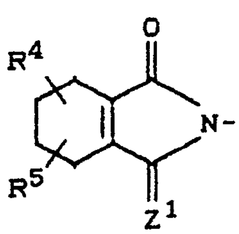 ; 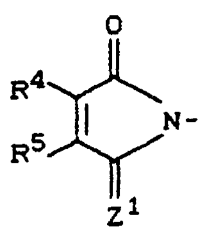 ; 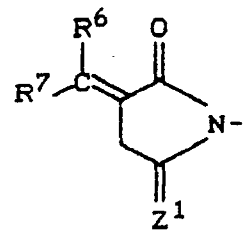

steht,
wenn man Anhydride der Formel (II),

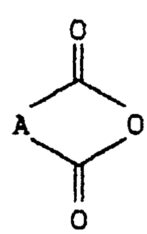

(II)

in welcher
A für einen Rest der Formel

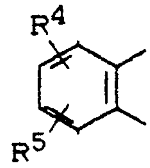 ; 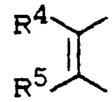 oder 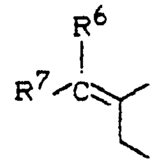

steht,
mit Aminobenzoxazinonen der Formel (III),

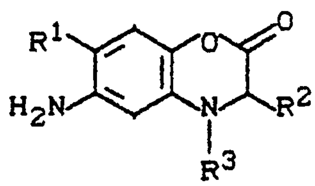 (III)

gegebenenfalls in Gegenwart eines Verdünnungsmittels und gegebenenfalls in Gegenwart eines Reaktionshilfsmittels umsetzt;
(b) man erhält N-Aryl-Stickstoffheterocyclen der Formel (Ib),

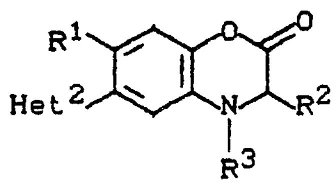 (Ib)

in welcher
$Het^2$ für einen Heterocyclus der Formel

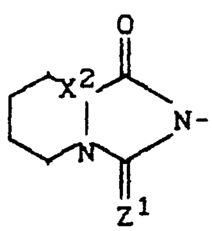 ; 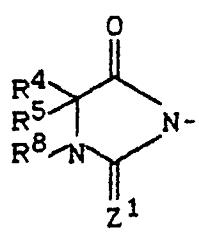 ; 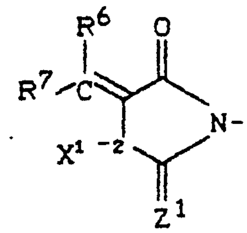

steht, wobei
$X^{1-2}$ für Sauerstoff oder eine

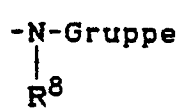

steht,
wenn man Carbonsäureester der Formel (IV),
$R^{15}$-$COOR^{16}$ (IV)
in welcher

$R^{15}$ für einen Rest der Formel

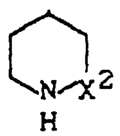 ; 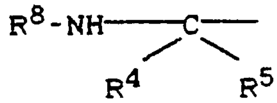 oder 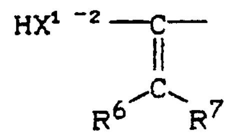

steht und
$R^{16}$ für Alkyl steht,
oder deren Säureadditionssalze
mit Iso(thio)cyanaten der Formel (V),

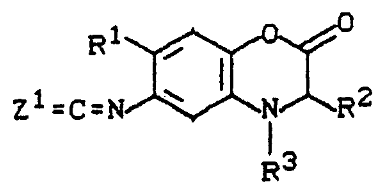

(V)

gegebenenfalls in Gegenwart eines Verdünnungsmittels und gegebenenfalls in Gegenwart eines Reaktionshilfsmittel, umsetzt;
(c) man erhält N-Aryl-Stickstoffheterocyclen der Formel (Ic),

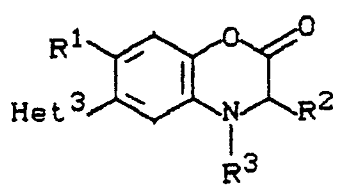

(Ic)

in welcher
$Het^3$ für einen Heterocyclus der Formel

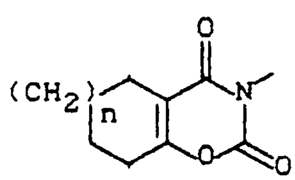 ; 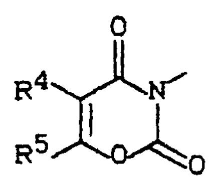

steht,
wenn man Verbindungen der Formeln (VIa) oder (VIb)

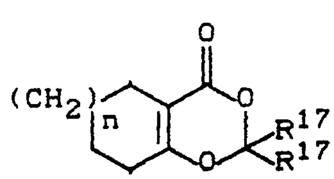 ; 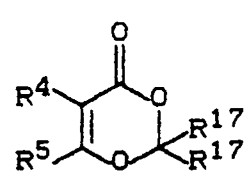

(VIa) (VIb)

in welcher
$R^{17}$ für Alkyl, vorzugsweise für $C_{1-4}$-Alkyl, insbesondere Methyl oder Ethyl steht,
mit Isocyanaten der Formel (Va)

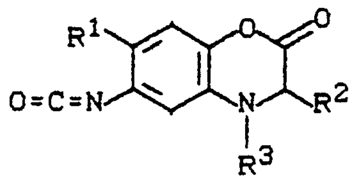

(Va)

in welcher
$R^3$ mit Ausnahme von Wasserstoff die oben angegebene Bedeutung hat,
gegebenenfalls in Gegenwart eines Verdünnungsmittels und gegebenenfalls in Gegenwart eines Reaktionshilfsmittels umsetzt;
(d) Man erhält N-Aryl-Stickstoffheterocyclen der Formel (Id)

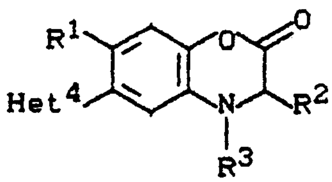

(Id)

in welcher
$Het^4$ für einen Heterocyclus der Formel

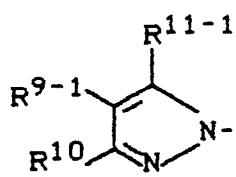

steht, wobei
$R^{9-1}$ für Wasserstoff, Alkyl oder Halogenalkyl oder gemeinsam mit $R^{10}$ für einen zweifach verknüpften Alkandiylrest steht und
$R^{11-1}$ für Wasserstoff, Alkyl oder Halogenalkyl steht und
$R^{10}$ die oben angegebene Bedeutung hat,
wenn man Phenylhydrazine der Formel (VII)

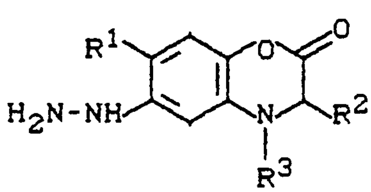

(VII)

mit 1,3-Diketonen der Formel (VIII),

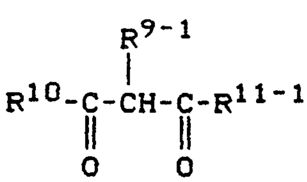

oder mit Derivaten dieser Diketone, wie beispielsweise Enolethern, Enolestern, Ketalen, Enoletherketalen, Enaminen oder $\beta$-Halogenvinylketonen gege benenfalls in Gegenwart eines Verdünnungsmittels und gegebenenfalls in Gegenwart eines Reaktionshilfsmittels umsetzt;

(e) man erhält N-Aryl-Stickstoffheterocyclen der Formel (Ie)

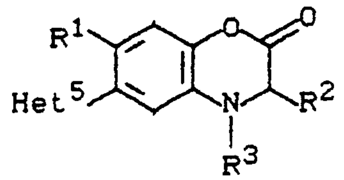

(Ie)

in welcher

$Het^5$ für einen Heterocyclus der Formel

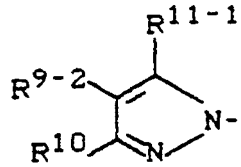

steht und

$R^{9-2}$ für Halogen steht,

wenn man N-Aryl-Stickstoffheterocyclen der Formel (If)

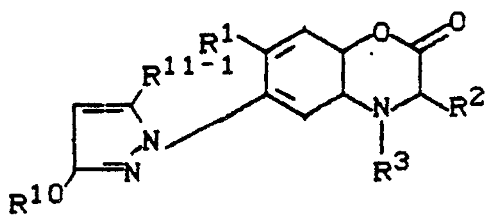

(If)

mit einem Halogenierungsmittel gegebenenfalls in Gegenwart eines Verdünnungsmittels umsetzt:

(f) man erhält N-Aryl-Stickstoffheterocyclen der Formel (Ig)

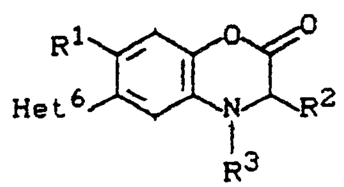

(Ig)

in welcher

$Het^6$ für einen Heterocyclus der Formel

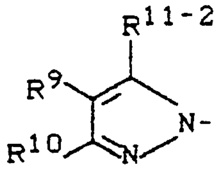

steht, wobei
$R^{11-2}$ für Halogen steht,
wenn man N-Aryl-pyrazolinone der Formel (IX),

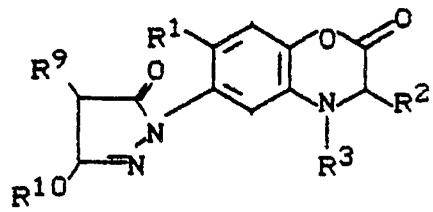

(IX)

mit einem Halogenierungsmittel, gegebenenfalls in Gegenwart eines Verdünnungsmittels und gegebenenfalls in Gegenwart eines Reaktionshilfsmittels umsetzt;
(g) man erhält N-Aryl-Stickstoffheterocyclen der Formel (Ih)

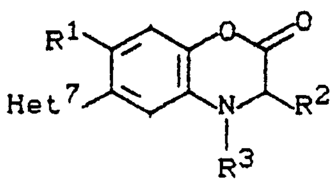

(Ih)

in welcher
$Het^7$ für einen Heterocyclus der Formel

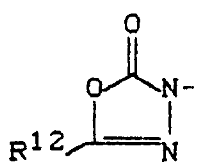

steht,
wenn man Phenylhydrazide der Formel (X),

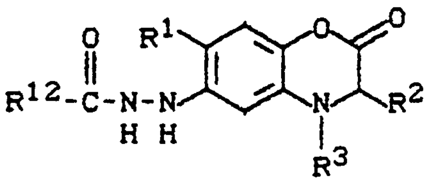

(X)

mit Phosgen gegebenenfalls in Gegenwart eines Verdünnungsmittels und gegebenenfalls in Gegenwart eines Reaktionshilfsmittels umsetzt;
(h) man erhält N-Aryl-Stickstoffheterocyclen der Formel (Ii)

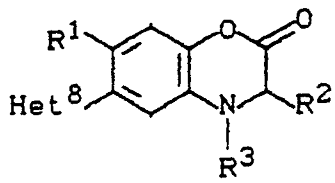

(Ii)

in welcher
Het[8] für einen Heterocyclus der Formel

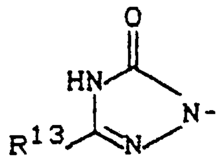

steht,
wenn man Phenylhydrazine der Formel (VII)

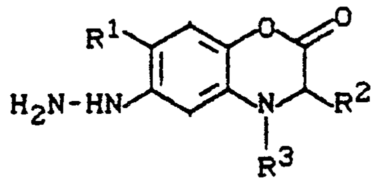

(VII)

mit Iminocarbonestern der Formel (XI)

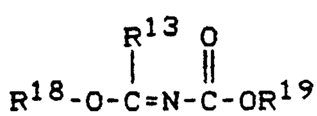

(XI)

in welcher
$R^{18}$ und $R^{19}$ unabhängig voneinander jeweils für Alkyl stehen,
gegebenenfalls in Gegenwart eines Verdünnungsmittels umsetzt;
(i) man erhält N-Aryl-Stickstoffheterocyclen der Formel (Ij)

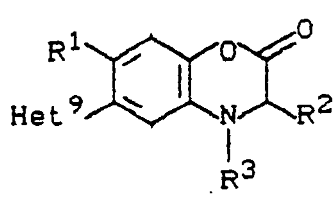

(Ij)

in welcher
Het[9] für einen Heterocyclus der Formel

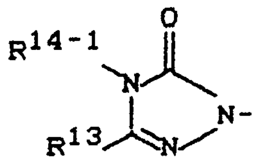

steht, in welcher
$R^{14-1}$ für Alkyl, Halogenalkyl, Alkenyl, Halogenalkenyl, Alkinyl oder Halogenalkinyl steht,
wenn man N-Aryl-Stickstoffheterocyclen der Formel (Ii)

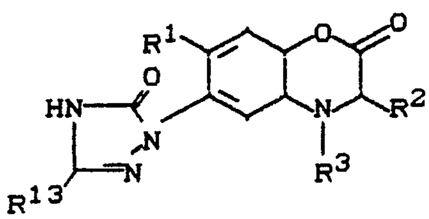

(Ii)

mit Alkylierungsmitteln der Formel (XII),
$R^{14-1}$-$E^1$ (XII)
in welcher
$R^{14-1}$ die oben angegebene Bedeutung hat und
$E^1$ für eine elektronenanziehende Abgangsgruppe steht,
gegebenenfalls in Gegenwart eines Verdünnungsmittels und gegebenenfalls in Gegenwart eines Reaktionshilfsmittels umsetzt;
(j) man erhält N-Aryl-Stickstoffheterocyclen der Formel (Ik)

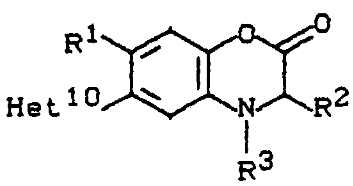

(Ik)

in welcher
$Het^{10}$ für einen Heterocyclus der Formel

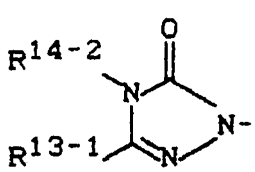

steht, wobei
$R^{13-1}$ und $R^{14-2}$ gemeinsam für einen zweifach verknüpften Alkandiylrest stehen,
wenn man Amidrazone der Formel (XIII)

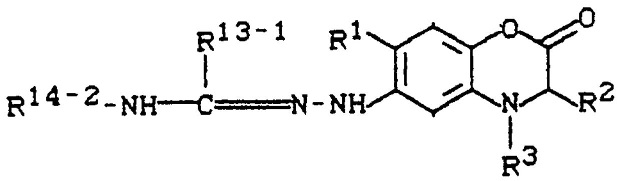

(XIII)

mit Phosgen gegebenenfalls in Gegenwart eines Verdünnungsmittels und gegebenenfalls in Gegenwart eines Reaktionshilfsmittels umsetzt;
(k) man erhält N-Aryl-Stickstoffheterocyclen der Formel (II)

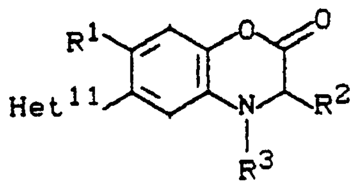

(Il)

in welcher
$Het^{11}$ für einen Heterocyclus der Formel

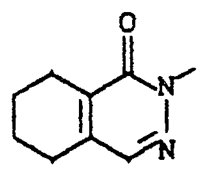

steht,
wenn man Hydroxyisobenzofuranon der Formel (XIV)

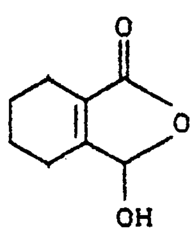

(XIV)

mit Phenylhydrazinen der Formel (VII)

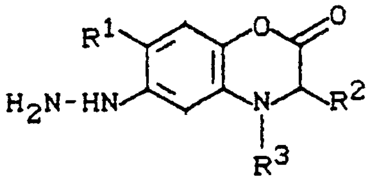

(VII)

gegebenenfalls in Gegenwart eines Verdünnungsmittels und gegebenenfalls in Gegenwart eines Reaktionshilfsmittels umsetzt;
(l) man erhält N-Aryl-Stickstoffheterocyclen der Formel (Im)

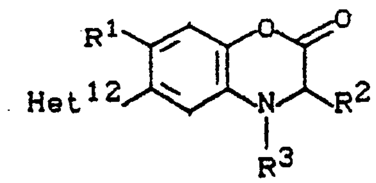

(Im)

in welcher
$Het^{12}$ für einen Heterocyclus der Formel

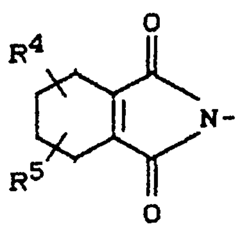 ; 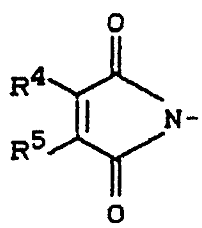 ; 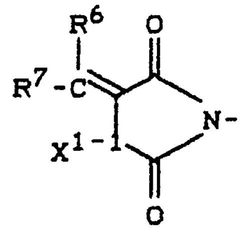 ;

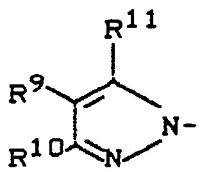 steht,

wenn man N-Aryl-Stickstoffheterocyclen der Formel (In)

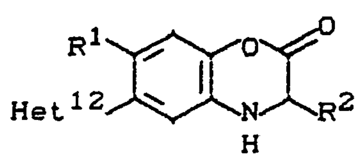

(In)

mit Alkylierungs- bzw. Acylierungsmitteln der Formel (XVI)
$R^3$-$E^2$ (XVI)
in welcher
$R^3$ mit Ausnahme von Wasserstoff die oben angegebene Bedeutung hat und
$E^2$ für einen elektronenanziehende Abgangsgruppe steht,
gegebenenfalls in Gegenwart eines Verdünnungsmittels und gegebenenfalls in Gegenwart eines Reaktionshilfsmittels umsetzt.

Schließlich wurde gefunden, daß die neuen N-Aryl-Stickstoffhetereocyclen der Formel (I) ausgezeichnete Nutzpflanzenselektivität und herbizide Eigenschaften besitzen.

In den Definitionen sind die gesättigten oder ungesättigten Kohlenwasserstoffketten wie beispielsweise Alkyl, Alkoxy, Alkylsulfonyl, Alkenyl, Alkinyl oder Cycloalkylalkyl, jeweils geradkettig oder verzweigt.

Halogen steht im allgemeinen für Fluor, Chlor, Brom oder Iod. Vorzugsweise steht Halogen für Fluor, Chlor oder Brom, insbesondere für Fluor oder Chlor.

Bevorzugt sind N-Aryl-Stickstoffheterocyclen der Formel (I), in welcher
Het für einen Heterocyclus der Formel steht

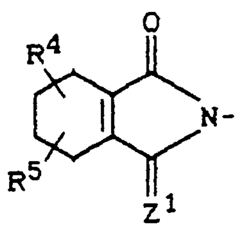 ; 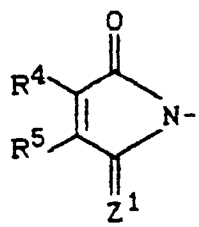 ; 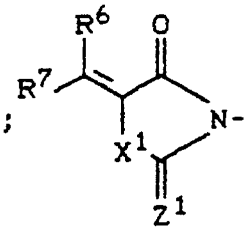 ;

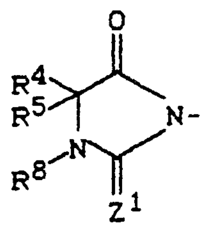 ; 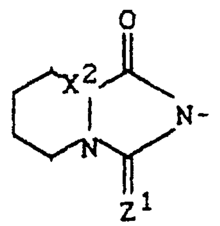 ; 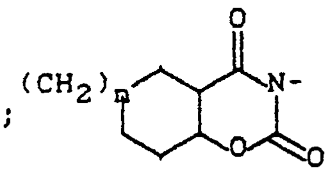 ;

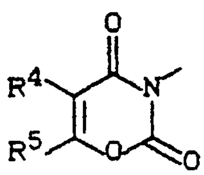 ; 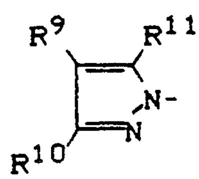 ; 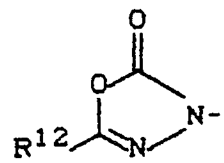 ;

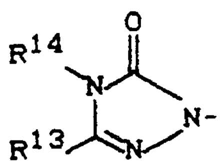 oder 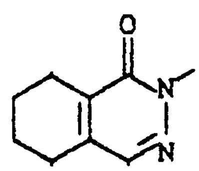

steht,
$R^1$ für Wasserstoff oder Halogen steht,
$R^2$ für Wasserstoff oder Alkyl mit 1 bis 4 Kohlenstoffatomen steht,
$R^3$ für Wasserstoff, Alkyl mit 1 bis 8 Kohlenstoffatomen, Alkenyl mit 2 bis 8 Kohlenstoffatomen, Alkinyl mit 3 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 3 bis 8 Kohlenstoffatomen und 1 bis 13 gleichen oder verschiedenen Halogenatomen, Alkylsulfonyl, Cyanalkyl, Alkoxyalkyl, Alkylthioalkyl, Halogenalkoxyalkyl, Alkoxyalkoxyalkyl, Bis-(Alkoxy)alkyl, Bis-(Alkylthio)alkyl, Alkylcarbonyl, Alkoxycarbonyl, Alkylcarbonylalkyl, Alkoxycarbonylalkyl oder Alkoxyalkoxycarbonylalkyl mit jeweils 1 bis 8 Kohlenstoffatomen in den einzelnen Alkylteilen und gegebenenfalls 1 bis 9 gleichen oder verschiedenen Halogenatomen oder für jeweils gegebenenfalls einfach oder mehrfach, gleich oder verschieden substituiertes Cycloalkyl, Cycloalkenyl, Cycloalkyloxycarbonylalkyl oder Cycloalkylalkyl mit jeweils 3 bis 7 Kohlenstoffatomen im Cycloalkylteil und gegebenenfalls 1 bis 4 Kohlenstoffatomen im Alkylteil steht, wobei als Substituenten im Cycloalkylteil jeweils in Frage kommen:
Halogen sowie Alkyl oder Alkoxy mit jeweils 1 bis 4 Kohlenstoffatomen, $R^3$ außerdem für jeweils gegebenenfalls durch Alkyl mit 1 bis 4 Kohlenstoffatomen substituiertes Oxetanylalkyl, Tetrahydrofuranylalkyl oder Tetrahydropyranylalkyl mit je weils 1 bis 3 Kohlenstoffatomen in den jeweiligen Alkylteilen steht und für jeweils gegebenenfalls einfach oder mehrfach gleich oder verschieden substituiertes Aralkyl mit 6 bis 10 Kohlenstoffatomen im Arylteil und 1 bis 4 Kohlenstoffatomen im Alkylteil oder für Arylsulfonyl mit 6 bis 10 Kohlenstoffatomen steht, wobei als Arylsubstituenten jeweils in Frage kommen: Halogen, Cyano, Nitro, Alkyl, Alkoxy, Alkylthio oder Alkoxycarbonyl mit jeweils 1 bis 4 Kohlenstoffatomen oder Halogenalkyl,

Halogenalkoxy oder Halogenalkylthio mit jeweils 1 bis 4 Kohlenstoffatomen und 1 bis 9 gleichen oder verschiedenen Halogenatomen,

$R^4$ und $R^5$ unabhängig voneinander jeweils für Wasserstoff oder Alkyl mit 1 bis 4 Kohlenstoffatomen stehen,

$R^6$ und $R^7$ entweder unabhängig voneinander jeweils für Wasserstoff oder für Alkyl mit 1 bis 4 Kohlenstoffatomen stehen oder gemeinsam für einen zweifachverknüpften Alkandiylrest mit 2 bis 7 Kohlenstoffatomen stehen

$R^8$ für Wasserstoff, Alkyl mit 1 bis 4 Kohlenstoffatomen oder für gegebenenfalls einfach oder mehrfach, gleich oder verschieden substituiertes Phenyl steht, wobei als Substituenten in Frage kommen: Halogen, Cyano, Nitro, Alkyl, Alkoxy oder Alkylthio mit jeweils 1 bis 4 Kohlenstoffatomen sowie Halogenalkyl, Halogenalkoxy oder Halogenalkylthio mit jeweils 1 bis 4 Kohlenstoffatomen und 1 bis 9 gleichen oder verschiedenen Halogenatomen und

$R^9$ für Wasserstoff, Fluor, Chlor, Brom, Iod, Alkyl mit 1 bis 6 Kohlenstoffatomen oder für Halogenalkyl mit 1 bis 4 Kohlenstoffatomen und 1 bis 9 gleichen oder verschiedenen Halogenatomen steht oder

$R^{10}$ für Wasserstoff, Alkyl mit 1 bis 6 Kohlenstoffatomen oder Halogenalkyl mit 1 bis 4 Kohlenstoffatomen und 1 bis 9 gleichen oder verschiedenen Halogenatomen steht oder

$R^9$ und $R^{10}$ gemeinsam für einen zweifach verknüpften Alkandiylrest mit 2 bis 6 Kohlenstoffatomen stehen,

$R^{11}$ für Wasserstoff, Fluor, Chlor, Brom, Iod, Alkyl mit 1 bis 6 Kohlenstoffatomen oder Halogenalkyl mit 1 bis 4 Kohlenstoffatomen und 1 bis 9 gleichen oder verschiedenen Halogenatomen steht,

$R^{12}$ für Wasserstoff, Alkyl mit 1 bis 6 Kohlenstoffatomen, Alkenyl mit 3 bis 6 Kohlenstoffatomen, Alkinyl mit 3 bis 6 Kohlenstoffatomen, Halogenalkyl mit 1 bis 4 Kohlenstoffatomen und 1 bis 9 gleichen oder verschiedenen Halogenatomen, Halogenalkenyl mit 3 bis 6 Kohlenstoffatomen und 1 bis 5 gleichen oder verschiedenen Halogenatomen, Halogenalkinyl mit 3 bis 6 Kohlenstoffatomen und 1 bis 5 gleichen oder verschiedenen Halogenatomen oder Alkoxyalkyl mit jeweils 1 bis 4 Kohlenstoffatomen in den einzelnen Alkylteilen steht oder für gegebenenfalls einfach oder mehrfach, gleich oder verschieden substituiertes Cycloalkyl mit 3 bis 7 Kohlenstoffatomen steht, wobei als Substituenten infrage kommen: Halogen, Alkyl oder Alkoxy mit jeweils 1 bis 4 Kohlenstoffatomen,

$R^{13}$ für Wasserstoff, Alkyl mit 1 bis 6 Kohlenstoffatomen, Alkenyl mit 3 bis 6 Kohlenstoffatomen, Alkinyl mit 3 bis 6 Kohlenstoffatomen, Halogenalkyl mit 1 bis 4 Kohlenstoffatomen und 1 bis 9 gleichen oder verschiedenen Halogenatomen, Halogenalkenyl mit 3 bis 6 Kohlenstoffatomen und 1 bis 5 gleichen oder verschiedenen Halogenatomen, Halogenalkinyl mit 3 bis 6 Kohlenstoffatomen und 1 bis 5 gleichen oder verschiedenen Halogenatomen oder Alkoxyalkyl mit jeweils 1 bis 4 Kohlenstoffatomen in den einzelnen Alkylteilen steht oder für gegebenenfalls einfach oder mehrfach, gleich oder verschieden substituiertes Cycloalkyl mit 3 bis 7 Kohlenstoffatomen steht, wobei als Substituenten infrage kommen: Halogen, Alkyl oder Alkoxy mit jeweils 1 bis 4 Kohlenstoffatomen und

$R^{14}$ für Wasserstoff, Alkyl mit 1 bis 6 Kohlenstoffatomen, Alkenyl mit 3 bis 6 Kohlenstoffatomen, Alkinyl mit 3 bis 6 Kohlenstoffatomen, Halogenalkyl mit 1 bis 4 Kohlenstoffatomen und 1 bis 9 gleichen oder verschiedenen Halogenatomen, Halogenalkenyl mit 3 bis 6 Kohlenstoffatomen und

1 bis 5 gleichen oder verschiedenen Halogenatomen oder Halogenalkinyl mit 3 bis 6 Kohlenstoffatomen und 1 bis 5 gleichen oder verschiedenen Halogenatomen steht oder

$R^{13}$ und $R^{14}$ gemeinsam für einen zweifach verknüpften Alkandiylrest mit 2 bis 6 Kohlenstoffatomen stehen

$X^1$ für Sauerstoff, eine $CH_2$-Gruppe oder eine

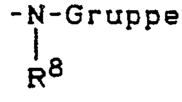

steht,

$X^2$ für Stickstoff oder eine CH-Gruppe steht,

$Z^1$ für Sauerstoff oder Schwefel steht und

n für die Zahl 0 oder 1 steht.

Besonders bevorzugt sind die N-Aryl-Stickstoffheterocyclen der Formel (I), in welcher

Het für einen Heterocyclus der Formel

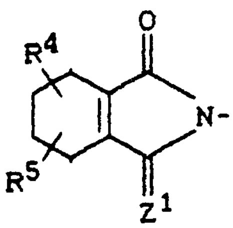 ; 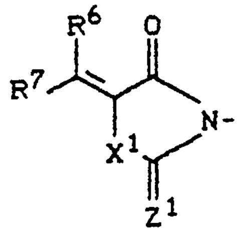 ; 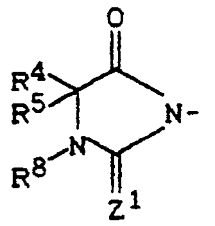 ;

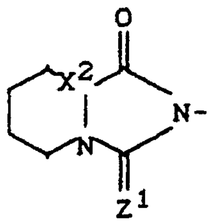 ; 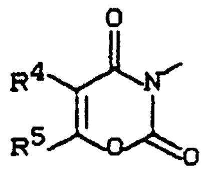 ; 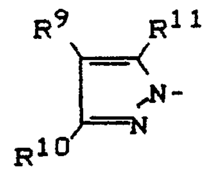 ;

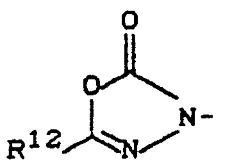 ; oder 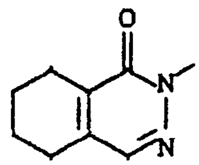

steht,
insbesondere für einen Heterocyclus der Formel

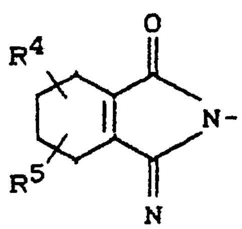 ; 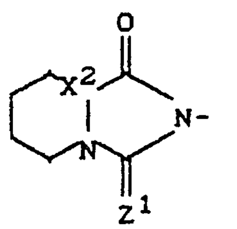 ; 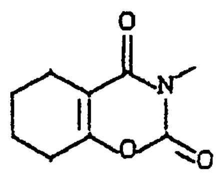

oder 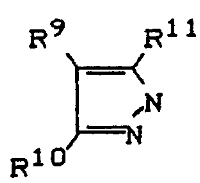 steht,

$R^1$ für Wasserstoff, Fluor, Chlor oder Brom steht,
$R^2$ für Wasserstoff, Methyl oder Ethyl steht,
$R^3$ für Methyl, Ethyl, n- oder i-Propyl, n-, i-, s-oder t-Butyl, für Allyl, Propargyl, für jeweils geradkettiges oder verzweigtes Pentyl, Hexyl, Butenyl, Pentenyl, Hexenyl, Butinyl, Pentinyl oder Hexinyl steht, außerdem für Halogenalkyl mit 1 bis 4 Kohlenstoffatomen oder Halogenalkenyl mit 3 bis 5 Kohlenstoffatomen und jeweils 1 bis 8 gleichen oder verschiedenen Halogenatomen, insbesondere Fluor, Chlor oder Brom steht, für Cyanalkyl, Alkoxyalkyl, Alkylthioalkyl, Alkylsulfonyl, Halogenalkoxyalkyl, Alkoxyalkoxyalkyl, Alkylcarbonyl, Alkoxycarbonyl, Alkylcarbonylalkyl, Alkoxycarbonylalkyl oder Alkoxyalkoxycarbonylalkyl mit jeweils 1 bis 5 Kohlenstoffatomen in den einzelnen Alkylteilen steht, außerdem für jeweils gegebenenfalls ein- bis dreifach, gleich und verschieden durch Methyl, Methoxy, Fluor oder Chlor substituiertes Cyclopropylmethyl, Cyclopentylmethyl, Cyclohexylmethyl, Cyclopropyloxycarbonylmethyl, Cyclopentyloxycarbonylmethyl, Cyclohexyloxycarbonylmethyl, Cyclopropyl, Cyclopentyl oder Cyclohexyl steht, für jeweils gegebenenfalls durch

Methyl und/oder Ethyl substituiertes Oxetanylmethyl, Oxetanylethyl, Tetrahydrofuranylmethyl, Tetrahydrofuranylethyl, Tetrahydropyranylmethyl oder Tetrahydropyranylethyl steht oder für jeweils gegebenenfalls ein- bis dreifach, gleich oder verschieden substituiertes Benzyl oder Phenylethyl steht, wobei als Substituenten jeweils in Frage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Methoxycarbonyl, Ethoxycarbonyl, Trifluormethyl, Trifluormethoxy oder Trifluormethylthio,

$R^4$ und $R^5$ unabhängig voneinander jeweils für Wasserstoff, Methyl, Ethyl, n- oder i-Propyl stehen,

$R^6$ und $R^7$ entweder unabhängig voneinander jeweils für Wasserstoff, Methyl, Ethyl, n- oder i-Propyl stehen oder gemeinsam für einen zweifach verknüpften Alkandiylrest mit 2 bis 5 Kohlenstoffatomen stehen,

$R^8$ für Wasserstoff, Methyl, Ethyl, n- oder i-Propyl oder für gegebenenfalls ein- bis dreifach, gleich oder verschieden substituiertes Phenyl steht, wobei als Substituenten in Frage kommen: Fluor, Chlor, Brom, Cyano, Nitro, Methyl, Ethyl, n- oder i-Propyl, n-, i-, s-oder t-Butyl, Methoxy, Ethoxy, n- oder i-Propoxy, Methylthio, Trifluormethyl, Trifluormethoxy oder Trifluormethylthio und

$R^9$ für Wasserstoff, Fluor, Chlor, Brom, Methyl, Ethyl, n- oder i-Propyl, n-, i-, s-oder t-Butyl, Fluormethyl, Difluormethyl, Trifluormethyl, Chlormethyl, Dichlormethyl, Trichlormethyl, Difluorchlormethyl oder Dichlorfluormethyl steht oder

$R^{10}$ für Wasserstoff, Methyl, Ethyl, n- oder i-Propyl, n-, i-, s- oder t-Butyl, Fluormethyl, Difluormethyl, Trifluormethyl, Chlormethyl, Dichlormethyl, Trichlormethyl, Difluorchlormethyl oder Dichlorfluormethyl steht und

$R^9$ und $R^{10}$ gemeinsam für einen 1,3-Propandiylrest einen 1,4-Butandiylrest oder einen 1,5-Pentandiylrest stehen,

$R^{11}$ für Wasserstoff, Fluor, Chlor, Brom, Methyl, Ethyl, n- oder i-Propyl, r-, i-, s-oder t-Butyl, Fluormethyl, Difluormethyl, Trifluormethyl, Chlormethyl, Dichlor-methyl, Trichlormethyl, Difluorchlormethyl oder Dichlorfluormethyl steht,

$R^{12}$ für Wasserstoff, Methyl, Ethyl, n- oder i-Propyl, n-, i-, s- oder t-Butyl, jeweils einfach, zweifach oder dreifach durch Fluor, und/oder Chlor substituiertes Methyl, Ethyl oder t-Butyl, für Allyl, n- oder i-Butenyl, Chlorallyl, Dichlorallyl, Propargyl, Chlorpropargyl, Methoxymethyl oder jeweils gegebenenfalls ein-bis fünffach, gleich oder verschieden durch Fluor, Chlor, Methyl und/oder Methoxy substituiertes Cyclopropyl, Cyclopentyl oder Cyclohexyl steht,

$X^1$ für Sauerstoff, eine $CH_2$-Gruppe oder eine

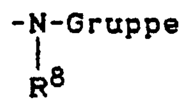

steht,

$X^2$ für Stickstoff oder eine CH-Gruppe steht, und

$Z^1$ für Sauerstoff oder Schwefel steht.

In allen Definitionsbereichen ist insbesondere die Bedeutung von

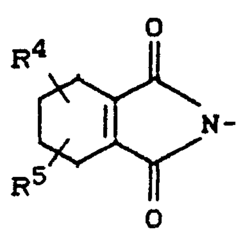

und

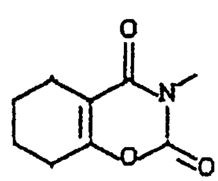

für Het hervorzuheben,

wobei $R^4$ und $R^5$ für Wasserstoff oder Methyl stehen.

In den allgemeinen Formeln steht $R^1$ vorzugsweise für Fluor.

In den allgemeinen Formeln steht $R^2$ vorzugsweise für Wasserstoff.

In den allgemeinen Formeln steht $R^3$ vorzugsweise für Wasserstoff, Propargyl, Cyanomethyl, Cyanoethyl, Methylsulfonyl, Ethylsulfonyl, Methoxycarbonyl oder Methoxyethoxycarbonyl.

Verwendet man beispielsweise 3,4,5,6-Tetrahydrophthalsäureanhydrid und 6-Amino-7-fluor-4-propargyl-3H-1,4-benzoxazin-2(4H)-on als Ausgangsstoffe, so läßt sich der Reaktionsablauf des erfindungsgemäßen Verfahrens (a) durch das folgende Formelschema darstellen:

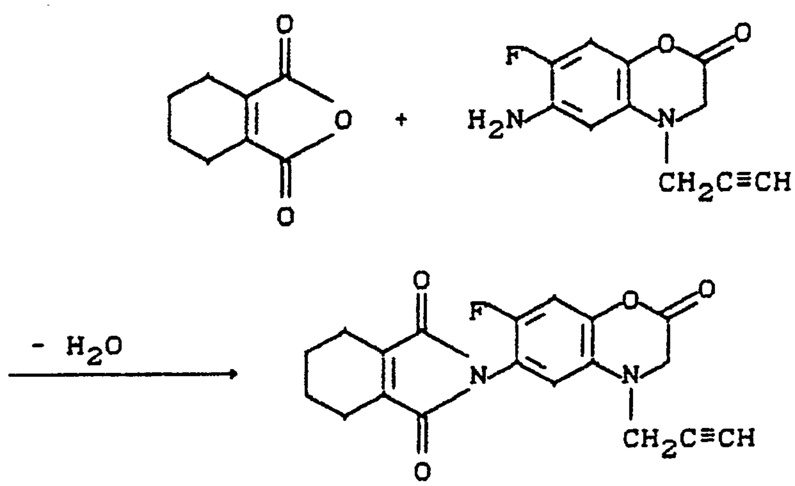


Verwendet man beispielsweise 2-Piperidinylcarbonsäureethylester und 4-Propargyl-3H-1,4-benzoxazin-2(4H)-on-6-yl-isocyanat als Ausgangsstoffe, so läßt sich der Reaktionsablauf des erfindungsgemäßen Verfahrens (b) durch das folgende Formelschema darstellen:

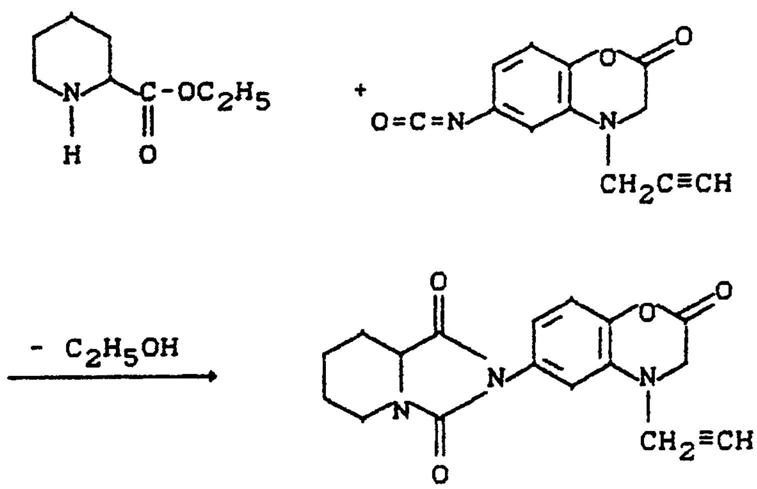


Verwendet man beispielsweise 2,2-Dimethyl-4,5,6,7,8-hexahydro-cyclohexa-1,3-dioxin-4-on und 4-Propargyl-3H-1,4-benzoxazin-2(4H)-on-6-yl-isocyanat als Ausgangsstoffe, so läßt sich der Reaktionsablauf des erfindungsgemäßen Verfahrens (c) durch das folgende Formelschema darstellen:

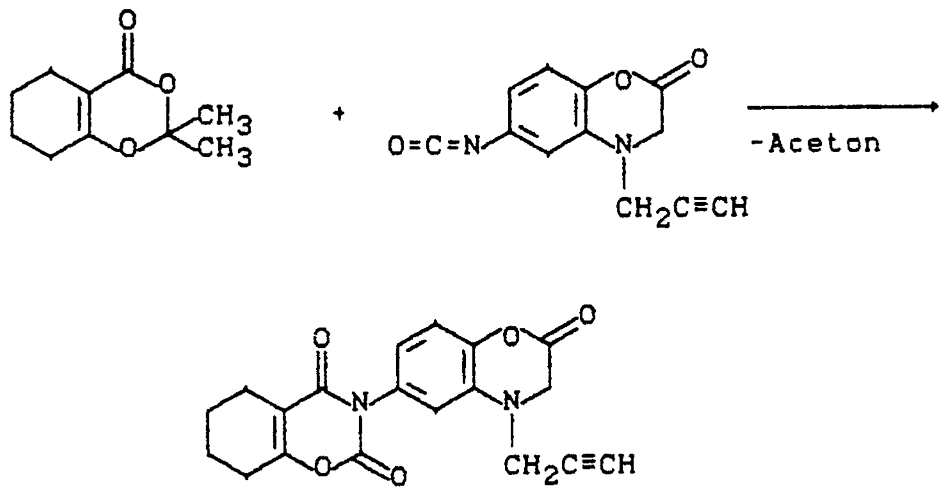


Verwendet man beispielsweise 7-Fluor-3H-1,4-benzoxazin-2(4H)-on-6yl-hydrazin und Acetylaceton als Ausgangsstoffe, so läßt sich der Reaktionsablauf des erfindungsgemäßen Verfahrens (d) durch das folgende Formelschema darstellen:

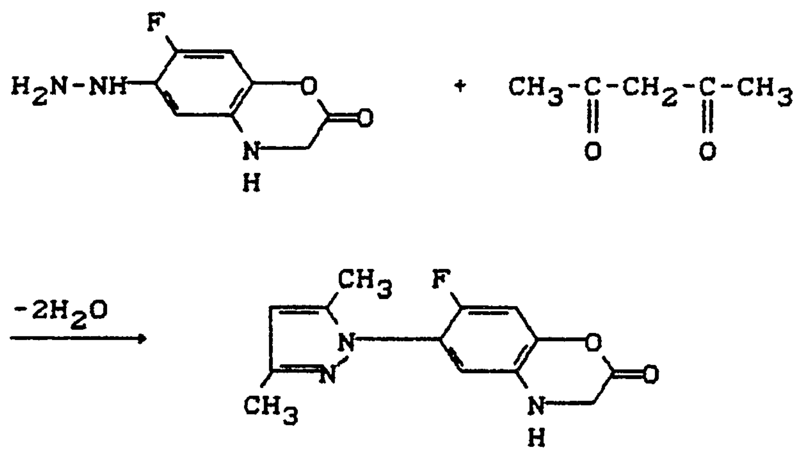


Verwendet man beispielsweise 6-(3,5-Dimethylpyrazol-1-yl)-4-propargyl-7-fluor-3H-1,4-benzoxazin-2-(4H)-on als Ausgangsverbindung und Sulfurylchlorid als Halogenierungsmittel, so läßt sich der Reaktionsablauf des erfindungsgemäßen Verfahrens (e) durch das folgende Formelschemȧ darstellen:

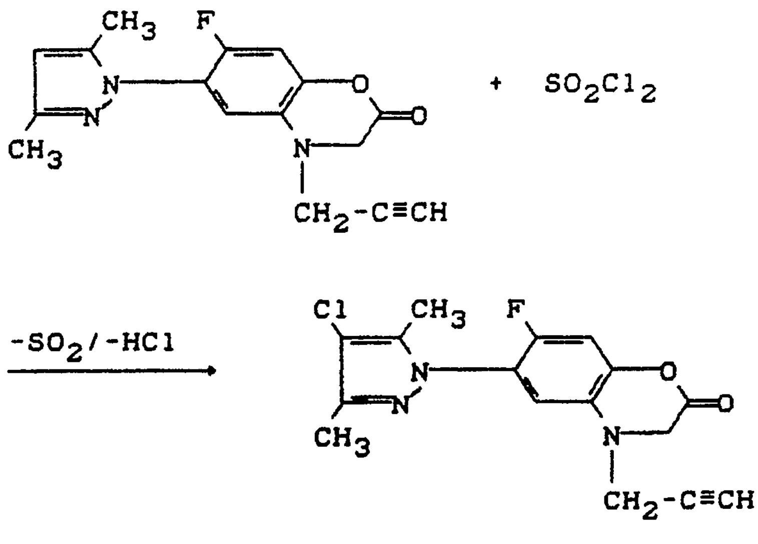


Verwendet man beispielsweise (7-Fluor-4-propargyl-3H-1,4-benzoxazin-2-(4H)-on-6-yl)-4H-pyrazolin-5-on als Ausgangsverbindung und Phosphoroxychlorid in Gegenwart von Triphenylphosphan als Halogenierungsmittel, so läßt sich der Reaktionsablauf des erfindungsgemäßen Verfahrens (f) durch das folgende Formelschema darstellen:

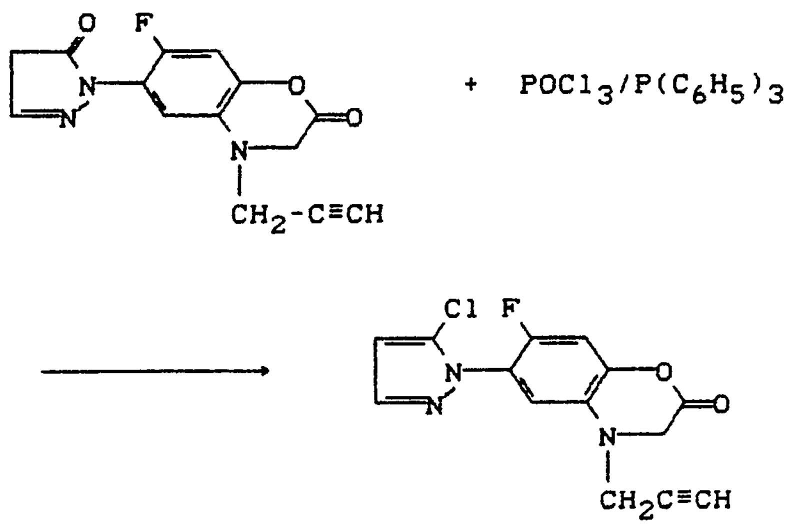


Verwendet man beispielsweise N-Pivaloyl-N′-(7-fluor-4-propargyl-3H-1,4-benzoxazin-2(4H)-on-6-yl)-hydrazin als Ausgangsverbindung, so läßt sich der Reaktionsablauf des erfindungsgemäßen Verfahrens (g) durch das folgende Formelschema darstellen:

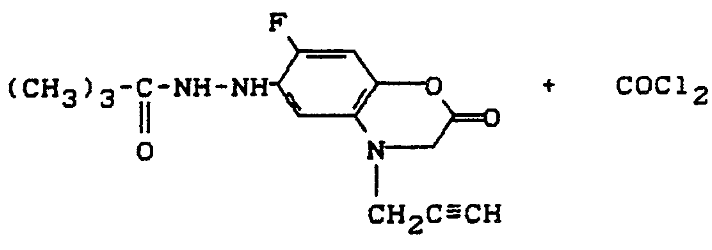

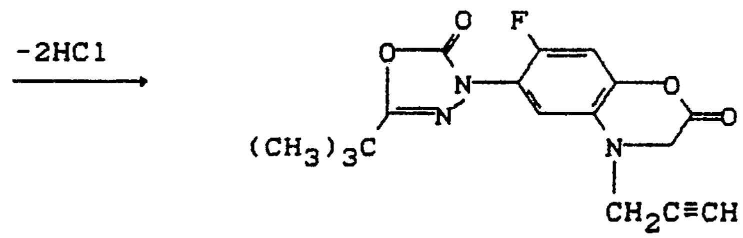


Verwendet man beispielsweise 7-Fluor-3H-1,4-benzoxazin-2(4H)-on-6-yl-hydrazin und N-Ethoxycarbonyl-2,2-dimethylpropanimidsäureethylester als Ausgangsstoffe, so läßt sich der Reaktionsablauf des erfindungsgemäßen Verfahrens (h) durch das folgende Formelschema darstellen:

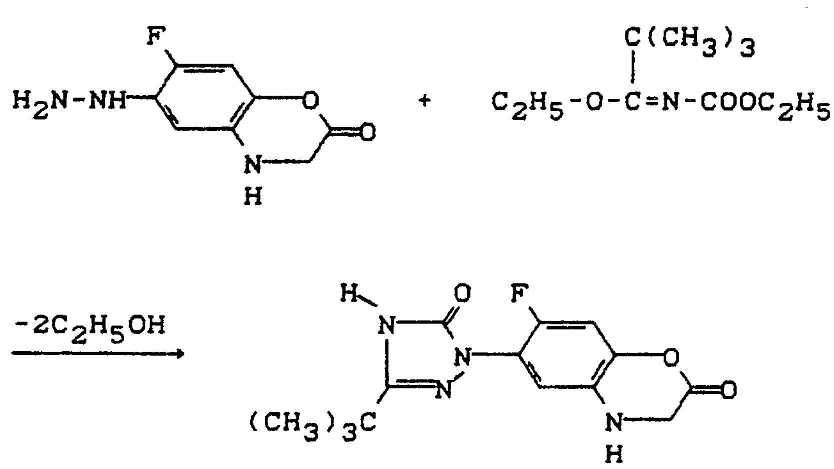


Verwendet man beispielsweise (7-Fluor-4-propargyl-3H-1,4-benzoxazin-2(4H)-on-6-yl)-3-t-butyl-4H-1,2,4-triazolin-5-on und Methyliodid als Ausgangsstoffe, so läßt sich der Reaktionsablauf des erfindungsgemäßen Verfahrens (i) durch das folgende Formelschema darstellen:

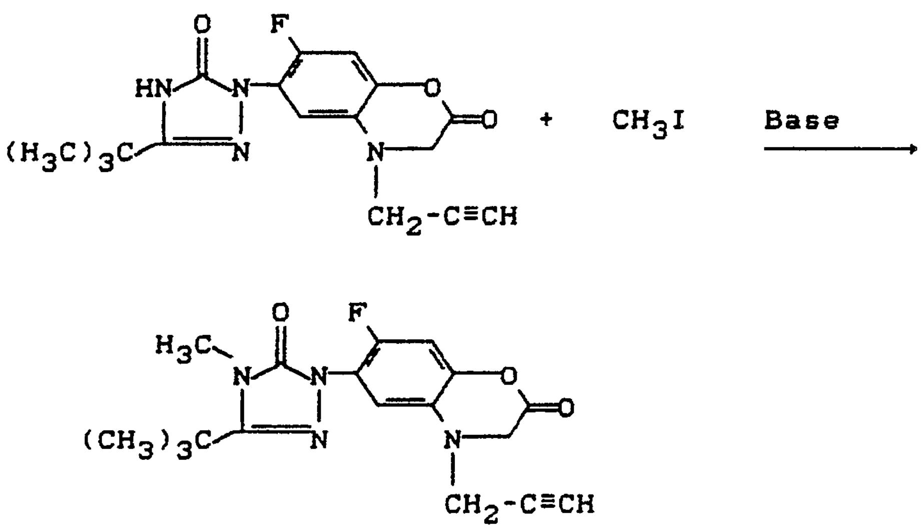


Verwendet man beispielsweise Piperidin-2-on-(7-fluor-4-methyl-3H-1,4-benzoxazin-2(4H)-on-6-yl-hydrazon als Ausgangsverbindung,so läßt sich der Reaktionsablauf des erfindungsgemäßen Verfahrens (j) durch das folgende Formelschema darstellen:

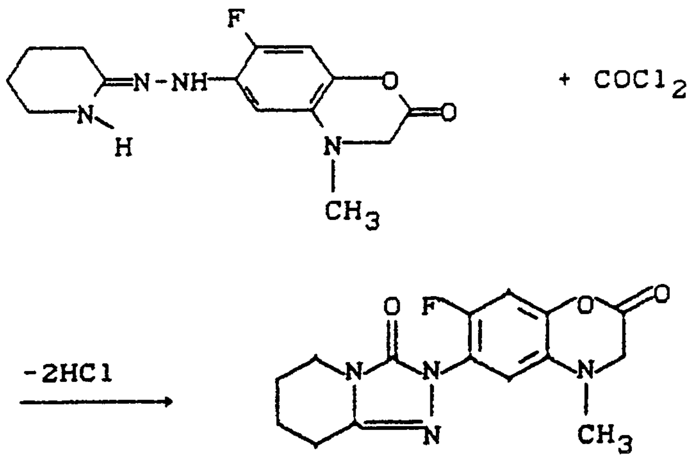


Verwendet man beispielsweise Tetrahydro-hydroxy-isobenzofuran und 4-Propargyl-3H-1,4-benzoxazin-2(4H)-on-6-yl-hydrazin als Ausgangsverbindungen, so läßt sich der Reaktionsablauf des erfindungsgemäßen Verfahrens (k) durch das folgende Formelschema darstellen:

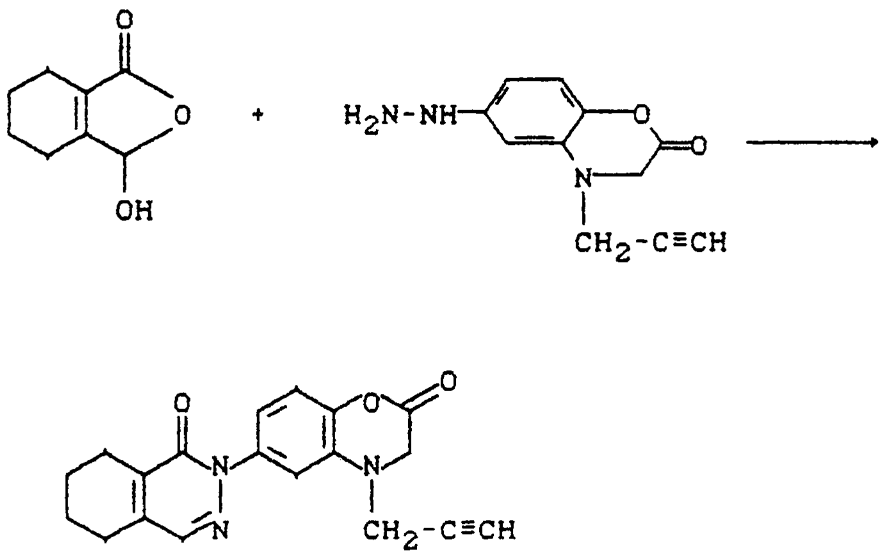

Verwendet man beispielsweise (7-Fluor-3H-1,4-benzoxazin-2(4H)-on-6-yl)-3-t-butyl-4-methyl-4H-1,2,4-triazolin-5-on und Propargylbromid als Ausgangsstoffe, so läßt sich der Reaktionsablauf des erfindungsgemäßen Verfahrens (l) durch das folgende Formelschema darstellen:

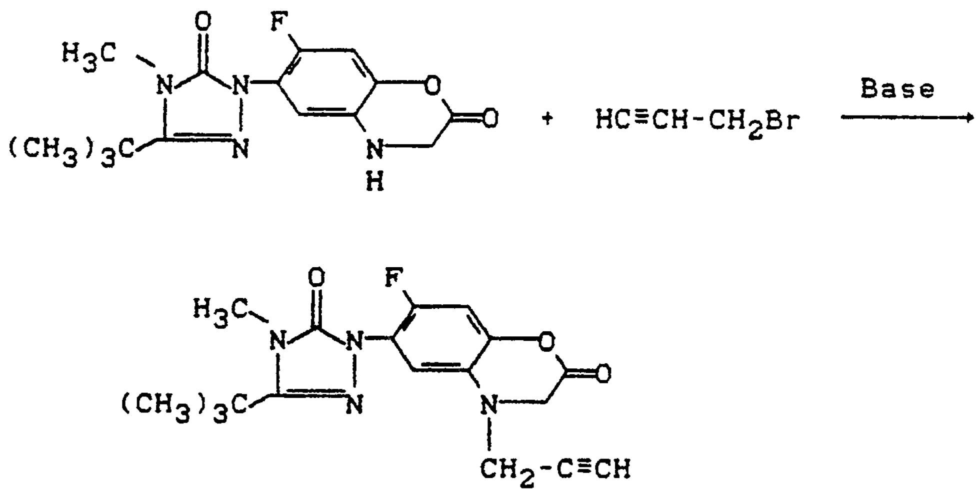


Die zur Durchführung des erfindungsgemäßen Verfahrens (a) als Ausgangsstoffe benötigten Anhydride der Formel (II) sind bekannt oder erhältlich in Analogie zu bekannten Verfahren (vgl. z.B. Gazz. chim. Ital. 57, 300-311 [1927]; DE-OS 3 644 222; J. Org. Chem. 51, 4150-4158 [1986]; Tetrahedron Lett. 25, 6027-6030 [1984]; J. Org. Chem. 42, 4162-4164 [1977]; Liebigs Ann. Chem. 1977, 772-790; Tetrahedron 25, 4099-4108 [1969]; JP 43/9046).

Die zur Durchführung des erfindungsgemäßen Verfahrens (a) weiterhin als Ausgangsstoffe benötigten Aminobenzoxazinone der Formel (III) sind noch nicht bekannt und ebenfalls Gegenstand der Erfindung.

Man erhält sie, wenn man 2-Nitrophenole der Formel (XVII)

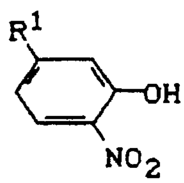

(XVII)

in welcher
$R^1$ für Wasserstoff oder Halogen steht,
in einer ersten Stufe mit Derivaten des Glykolsäurechlorids der allgemeinen Formel

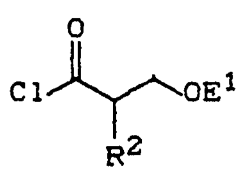

(XVIII)

in welcher
$E^1$ für eine elektronenziehende Abgangsgruppe steht, in Gegenwart eines Verdünnungsmittels wie beispielsweise Toluol und gegebenenfalls in Gegenwart eines Reaktionshilfsmittels wie beispielsweise Triethylamin bei Temperaturen zwischen -30° C und 120° C umsetzt und die so erhältlichen Glykolsäureester der Formel (XIX)

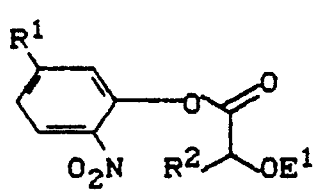

(XIX)

in einer 2. Stufe entweder mit molekularem Wasserstoff in Gegenwart eines Edelmetallkatalysators wie z.B. Pd/C in einem Verdünnungsmittel wie beispielsweise Tetrahydrofuran reduziert und anschließend säurekatalysiert cyclisiert, oder direkt in verdünnter Säure, wie beispielsewise Essigsäure, bei Temperaturen zwischen 20° und 150° C reduktiv cyclisiert.

Die so erhaltenen Benzoxazinone der Formel (XX)

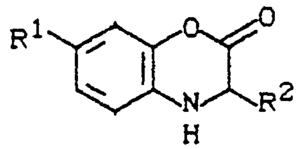

(XX)

werden dann in üblicher Art und Weise mit Salpetersäure in Schwefelsäure nitriert und anschließend ebenfalls in allgemein üblicher Art und Weise mit einem Reaktionsmittel wie beispielsweise Eisen in Essigsäure reduziert.

Die zur Durchführung des Verfahrens (b) als Ausgangsstoffe benötigten Carbonsäureester der Formel (IV) sind allgemein bekannte Verbindungen der organischen Chemie oder erhältlich in Analogie zu bekannten Verfahren (vgl. z.B. US 4 730 006. Pestic. Sci. 16, 277-286 [1985]; Chem. Pharm. Bull. 32, 3934-3944 [1984]; Liebigs Ann. Chem. 1983, 1133-1151; Synthesis 1981, 915-917; Tetrahedron Lett. 22, 2485-2486 [1981]; Chem. Ber. 111, 1058-1076 [1978]; Angew. Chem. 89, 344-345 [1977]; Chem. Ber. 110, 942-947 [1977]; Chem. Lett. 1976, 1095-1096; Angew. Chem. 88, 295-296 [1976]; DE 2 058 012; Bull. chem. Soc. Japan 44, 474-477 [1971]; J. chem. Soc. Perkin I, 1987, 877-884; DE 3 702 943; DE 2 331 549; J. Heterocycl. Chem. 6, 181-185, [1969]).

In dieser Formel (IV) steht $R^{15}$ vorzugsweise für einen Rest der Formel

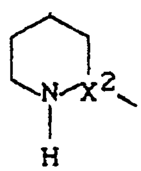 ; 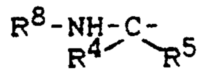 oder 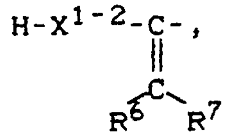,

wobei

$X^{1-2}$ für Sauerstoff oder eine

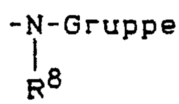

steht und

$R^{16}$ vorzugsweise für geradkettiges oder verzweigtes Alkyl mit 1 bis 4 Kohlenstoffatomen, insbesondere für Methyl oder Ethyl steht.

Die zur Durchführung des Verfahrens (b) bzw. (c) weiterhin benötigten Iso(thio)cyanate der Formel (V) bzw. (Va) sind erhältlich in Analogie zu bekannten Verfahren (vgl. z.B. FR 2 003 438; ZA 6713761 bzw. CA70: 67 955d; EP-A 105 991; EP-A 67 689 oder GB 1 336 871 sowie die Herstellungsbeispiele), beispielsweise wenn man Amine der Formel (III),

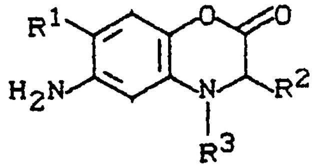

(III)

in welcher

$R^3$ mit Ausnahme von Wasserstoff die oben angegebene Bedeutung haben,

mit Phosgen, Thiophosgen oder Diphosgen ($Cl_3C$-O-CO-Cl) gegebenenfalls in Gegenwart eines Verdünnungsmittels wie beispielsweise Toluol bei Temperaturen zwischen 20 °C und 120 °C umsetzt.

Die zur Durchführung des Verfahrens (c) als Ausgangsstoffe benötigten Verbindungen der Formel (VIa) oder (VIb) sind allgemein bekannte Verbindungen und können nach an sich bekannten Verfahren hergestellt werden (vgl. Chem. Ber. 105 (1972), 137-149; J. Am. Chem. Soc. 74 (1952), 6305-6406; loc. cit. 75 (1953), 5400-5402; DE-OS 1 957 312).

Die zur Durchführung der Verfahren (d), (h) und (k) als Ausgangsstoffe benötigten Phenylhydrazine der Formel (VII) sind noch nicht bekannt und ebenfalls Gegenstand der vorliegenden Erfindung.

In der Formel (VII) stehen $R^{9-1}$ und $R^{10-1}$ unabhängig voneinander vorzugsweise für Wasserstoff, $C_1$-$C_6$-Alkyl oder Halogen-$C_1$-$C_4$-alkyl, insbesondere für Wasserstoff, Methyl, Ethyl, n- oder iso-Propyl, n-, iso-, sec.- oder tert-Butyl oder Halogenmethyl mit 1 bis 3 gleichen oder verschiedenen Halogenatomen oder $R^{9-1}$ und $R^{10-1}$ stehen gemeinsam für einen zweifach verknüpften Alkandiylrest mit vorzugsweise 2 bis 6 Kohlenstoffatomen, insbesondere für einen 1,3-Propandiylrest, einen 1,4-Butandiylrest oder einen 1,5-Pentandiylrest.

Man erhält die Phenylhydrazine der Formel (VII), wenn man Amino-benzoxazinone der Formel (III)

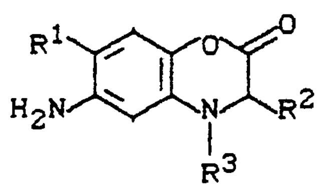

(III)

zunächst in üblicher Art und Weise mit Natriumnitrit in Gegenwart einer Säure wie beispielsweise Salzsäure diazotiert und anschließend ebenfalls in allgemein üblicher Art und Weise mit einem Reduktionsmittel wie beispielsweise Zinn(II)chlorid reduziert.

Die Verbindungen der Formel (III) und (VII) lassen sich in einer gemeinsamen Formel (IIIa) zusammenfassen:

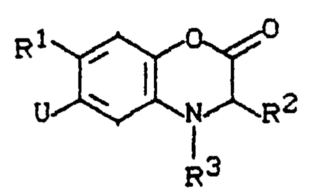

(IIIa)

in welcher

U für $-NH_2$ oder $-NH-NH_2$ steht.

Die zur Durchführung des Verfahrens (d) weiterhin als Ausgangsstoffe benötigten 1,3-Diketone der Formel (VIII) und Derivate dieser Diketone wie beispielsweise Enolether, Enolester, Ketale, Enoletherketale, Enamine oder β-Halogenvinylketone sind allgemein bekannte Verbindungen der organischen Chemie.

In der Formel (VIII) steht $R^{11-1}$ vorzugsweise für Wasserstoff, $C_1$-$C_6$-Alkyl oder Halogen-$C_1$-$C_4$-alkyl, insbesondere für Wasserstoff, Methyl, Ethyl, n- oder iso-Propyl, n-, iso-, sec.- oder tert.-Butyl oder Halogenmethyl mit 1 bis 3 gleichen oder verschiedenen Halogenatomen, insbesondere Fluor oder Chlor.

Die zur Durchführung des Verfahrens (e) als Ausgangsstoffe benötigten N-Aryl-Stickstoffheterocyclen der Formel (If) sind erfindungsgemäße Verbindungen und erhältlich mit Hilfe der erfindungsgemäßen Verfahren (d) und (l).

Die zur Durchführung des erfindungsgemäßen Verfahrens (f) als Ausgangsstoffe benötigten N-Arylpyrazolinone der Formel (IX) sind noch nicht bekannt und ebenfalls Gegenstand der vorliegenden Erfindung.

Man erhält sie, wenn man β-Ketoester der Formel (XXI)

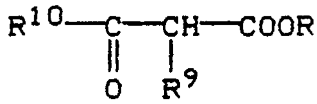

(XXI)

in welcher
R für Alkyl, insbesondere für Methyl oder Ethyl steht,
mit Phenylhydrazinen der Formel (VII),

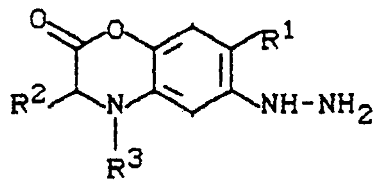

(VII)

gegebenenfalls in Gegenwart eines Verdünnungsmittels wie beispielsweise Ethanol und gegebenenfalls in Gegenwart eines Reaktionshilfsmittels wie beispielsweise Schwefelsäure bei Temperaturen zwischen 20° C und 120° C umsetzt.

Die β-Ketoester der Formel (XXI) sind allgemein bekannte Verbindungen der organischen Chemie.

Die zur Durchführung des Verfahrens (g) als Ausgangsstoffe benötigten Phenylhydrazide der Formel (X) sind noch nicht bekannt und ebenfalls Gegenstand der vorliegenden Erfindung.

Man erhält sie, wenn man Phenylhydrazine, der Formel (VII),

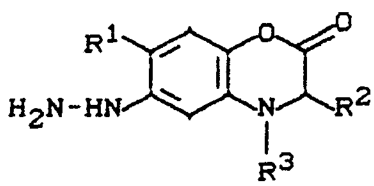

(VII)

mit Acylierungsmitteln der Formel (XXII),

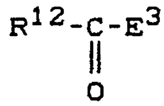

(XXII)

in welcher
$E^3$ für eine elektronenanziehende Abgangsgruppe, vorzugsweise für Halogen oder für einen Rest

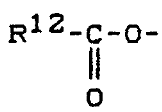

steht,

wobei
$R^{12}$ die oben angegebene Bedeutung hat und insbesondere für Chlor steht,
gegebenenfalls in Gegenwart eines Verdünnungsmittels wie beispielsweise Dichlormethan und gegebenenfalls in Gegenwart eines Reaktionshilfsmittels wie beispielsweise Triethylamin bei Temperaturenzwischen -20° C und +60° C umsetzt.

Acylierungsmittel der Formel (XXII) sind allgemein bekannte Verbindungen der organischen Chemie.

Die zur Durchführung des Verfahrens (h) als Ausgangsstoffe benötigten Iminocarbonester der Formel (XI) sind bekannt oder erhältlich in Analogie zu bekannten Verfahren (vgl. z.B. Chem. Ber. 119, 2444-2457 [1986]; Bull. chem. Soc. Jpn. 55, 3943-3944 [1982]; Chem. Lett. 1982, 1015-1016; Chem. Lett. 1978, 1403-1404; J. Amer. Chem. Soc. 95, 3957-3963 [1973]; J. Org. Chem. 36, 3251-3252 [1971]).

In Formel (XI) stehen $R^{18}$ und $R^{19}$ unabhängig voneinander vorzugsweise für $C_1$-$C_4$-Alkyl, insbesondere für Methyl oder Ethyl.

Die zur Durchführung des Verfahrens (i) als Ausgangsstoffe benötigten N-Aryl-Stickstoffheterocyclen der Formel (Ii) sind erfindungsgemäße Verbindungen und erhältlich mit Hilfe des erfindungsgemäßen Verfahrens (h).

Die weiterhin als Ausgangsstoffe benötigten Alkylierungsmittel der Formel (XII) sind allgemein bekannte Verbindungen der organischen Chemie.

In Formel (XII) steht $R^{14-1}$ vorzugsweise für $C_1$-$C_6$-Alkyl, $C_3$-$C_6$-Alkenyl, $C_3$-$C_6$-Alkinyl, Halogen-$C_1$-$C_4$-alkyl mit 1 bis 9 gleichen oder verschiedenen Halogenatomen, Halogen-$C_3$-$C_6$-alkenyl mit 1 bis 5 gleichen oder verschiedenen Halogenatomen oder Halogen-$C_3$-$C_6$-alkinyl mit 1 bis 5 gleichen oder verschiedenen Halogenatomen. $R^{14-1}$ steht insbesondere für Methyl, Ethyl, n- oder i-Propyl, n-, i-, s- oder t-Butyl, für jeweils einfach, zweifach oder dreifach durch Fluor und/oder Chlor substituiertes Methyl, Ethyl oder t-Butyl, für Allyl, n- oder i-Butenyl, Chlorallyl, Dichlorallyl, Propargyl oder Chlorpropargyl.

$E^1$ steht vorzugsweise für Halogen, insbesondere für Chlor, Brom oder Iod, oder für jeweils gegebenenfalls substituiertes Alkylsulfonyloxy, Alkoxysulfonyloxy oder Arylsulfonyloxy, wie beispielsweise Methansulfonyloxy, Trifluormethansulfonyloxy, Methoxysulfonyloxy, Ethoxysulfonyloxy oder p-Toluolsulfonyloxy.

Die zur Durchführung des erfindungsgemäßen Verfahrens (j) als Ausgangsstoffe benötigten Amidrazone der Formel (XIII) sind noch nicht bekannt.

$R^{13-1}$ und $R^{14-2}$ stehen vorzugsweise gemeinsam für einen zweifach verknüpften Alkandiylrest mit 2 bis 6 Kohlenstoffatomen, insbesondere für einen 1,3-Propandiylrest, einen 1,4-Butandiylrest oder einen 1,5-Pentandiylrest.

Man erhält die Amidrazone der Formel (XIII) in Analogie zu bekannten Verfahren (vgl. z.B. US-P 4 080 192 oder DE-OS 1 957 783), wenn man Lactame der Formel (XXIII)

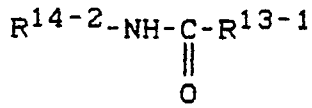

(XXIII)

zunächst in einer 1. Stufe mit einem Halogenierungsmittel wie beispielsweise Phosphoroxychlorid, Thionylchlorid oder Phosgen gegebenenfalls in Gegenwart eines Verdünnungsmittels wie beispielsweise Ethanol oder Dioxan und gegebenenfalls in Gegenwart eines Reaktionshilfsmittels wie beispielsweise Triethylamin oder Pyridin bei Temperaturen zwischen 0 °C und 50 °C umsetzt und anschließend die so erhältlichen Imidchloride der Formel (XXIV),

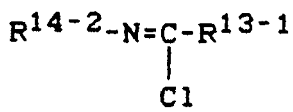

(XXIV)

mit Phenylhydrazinen der Formel (VII),

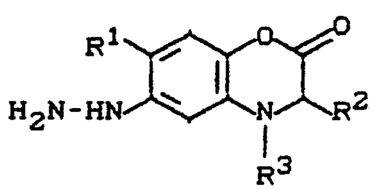

(VII)

gegebenenfalls in Gegenwart eines Verdünnungsmittels wie beispielsweise Ethanol und gegebenenfalls in Gegenwart eines Reaktionshilfsmittels wie beispielsweise Triethylamin oder Pyridin bei Temperaturen zwischen 0 °C und 80 °C umsetzt.

Lactame der Formel (XXIII) sind allgemein bekannte Verbindungen der organischen Chemie.

Das zur Durchführung des erfindungsgemäßen Verfahrens (k) als Ausgangsstoff benötigte Hydroxyisobenzfuranon der Formel (XIV) ist eine allgemein bekannte Verbindung der organischen Chemie.

Die zur Durchführung des Verfahrens (l) als Ausgangsstoffe benötigten N-Aryl-Stickstoffheterocyclen

der Formel (In) sind erfindungsgemäße Verbindungen und erhältlich mit Hilfe der erfindungsgemäßen Verfahren (a) bis (k).

Die weiterhin benötigten Alkylierungs- bzw. Acylierungsmittel der Formel (XVI) sind bekannte Ver bindungen der organischen Chemie. $E^2$ steht vorzugsweise für Halogen, insbesondere für Chlor, Brom oder Iod,oder für jeweils gegebenenfalls substituiertes Alkylsulfonyloxy mit vorzugsweise 1 bis 4 Kohlenstoffatomen, Alkoxysulfonyloxy mit vorzugsweise 1 bis 4 Kohlenstoffatomen oder Arylsulfonyloxy mit vorzugsweise 6 bis 10 Kohlenstoffatomen, wie insbesondere Methansulfonyloxy, Trifluormethansulfonyloxy, Methoxysulfonyloxy, Ethoxysulfonyloxy oder p-Toluolsulfonyloxy.

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyloder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester, Sulfoxide, wie Dimethylsulfoxid oder Carbonsäuren, wie Essigsäure oder Propionsäure.

Das erfindungsgemäße Verfahren (a) kann gegebenenfalls in Gegenwart eines geeigneten Reaktionshilfsmittels durchgeführt werden. Vorzugsweise verwendet man anorganische oder organische Säuren wie beispielsweise Essigsäure oder p-Toluolsulfonsäure, Anhydride wie beispielsweise Acetanhydrid oder Säurechloride wie Acetylchlorid als Reaktionshilfsmittel. Es ist auch möglich andere übliche wasserabspaltende Mittel wie beispielsweise N,N′-Dicyclohexylcarbodiimid oder übliche Acylierungskatalysatoren, wie beispielsweise 4-(N,N-Dimethylamino)-pyridin als Reaktionshilfsmittel zu verwenden.

Die Reaktionstemperaturen können bei der Durchführung des erfindungsgemäßen Verfahrens (a) in einem größeren Bereich variiert werden. Im allgemeinen arbeitet man bei Temperaturen zwischen 20 °C und 180 °C, vorzugsweise bei Temperaturen zwischen 50 °C und 150 °C.

Zur Durchführung des erfindungsgemäßen Verfahrens (a) setzt man pro Mol an Anhydrid der Formel (II) im allgemeinen 1 bis 1.5 Mol, vorzugsweise 1 bis 1.2 Mol an Aminobenzoxazinon der Formel (III) und gegebenenfalls 0.01 bis 1.2 Mol, vorzugsweise 0.1 bis 1 Mol an Reaktionshilfsmittel ein. Die Reaktionsdurchführung, Aufarbeitung und Isolierung der Reaktionsprodukte erfolgt nach allgemein üblichen Methoden (vgl. auch die Herstellungsbeispiele).

Als Verdünnungsmittel zur Durchführung des erfindungsgemäßen Verfahren (b) kommen inerte organische Lösungsmittel infrage. Hierzu gehören insbesondere ali phatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid oder Alkohole wie Methanol, Ethanol oder Propanol.

Das erfindungsgemäße Verfahren (b) wird gegebenenfalls in Gegenwart eines geeigneten Reaktionshilfsmittels durchgeführt. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (b) in einem größeren Bereich variiert werden. Im allgemeinen arbeitet man bei Temperaturen zwischen 0 °C und 150 °C, vorzugsweise bei Temperaturen zwischen 20 °C und 120 °C.

Zur Durchführung des erfindungsgemäßen Verfahrens (b) setzt man pro Mol an Carbonsäureester der Formel (IV) oder eines entsprechenden Säureadditionssalzes im allgemeinen 0.5 bis 5 Mol, vorzugsweise 0.8 bis 1.5 Mol an Iso(thio)cyanat der Formel (V) und gegebenenfalls 1 bis 10 Mol, vorzugsweise 1 bis 5 Mol an Reaktionshilfsmittel ein. Dabei ist es auch möglich die Iso(thio)cyanate der Formel (V) in einer vorgelagerten Reaktion aus Aminen der Formel (III) und Phosgen, Thiophosgen oder Diphosgen ($Cl_3C$-O-CO-Cl) direkt im Reaktionsgefäß herstellen und ohne Isolierung im "Eintopfverfahren" mit den Carbonsäureestern der Formel (IV) weiter umzusetzen.

Die Reaktionsdurchführung, Aufarbeitung und Isolierung der Reaktionsprodukte erfolgt nach allgemein üblichen Methoden (vgl. auch die Herstellungsbeispiele).

Das erfindungsgemäße Verfahren (c) wird gegebenenfalls unter Verwendung von Verdünnungsmitteln durchgeführt. Als Verdünnungsmittel kommen vorzugsweise hochsiedende, inerte organische Lösungsmittel in Betracht. Hierzu gehören vor allem, gegebenenfalls halogenierte, Kohlenwasserstoffe, wie Dekalin,

Tetralin, Toluol, Xylol, ferner Chlorbenzol und 1,2-Dichlorbenzol sowie Mesitylen.

Die Reaktionstemperaturen können bei dem erfindungsgemäßen Verfahren (c) in einem größeren Bereich variiert werden. Im allgemeinen arbeitet man bei Temperaturen zwischen 80 °C und 250 °C, vorzugsweise bei Temperaturen zwischen 120 °C und 220 °C.

Das erfindungsgemäße Verfahren (c) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (c) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel - gegebenenfalls aber auch ohne Verdünnungsmittel - durchgeführt und das Reaktionsgemisch wird bei der jeweils erforderlichen Temperatur gerührt. Die Aufarbeitung erfolgt bei dem erfindungsgemäßen Verfahren (c) jeweils nach üblichen Methoden.

Als Verdünnungsmittel zur Durchführung der erfindungsgemäßen Verfahren (d) und (k) kommen inerte organische Lösungsmittel infrage. Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyloder diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester, Sulfoxide, wie Dimethylsulfoxid, Alkohole wie Methanol, Ethanol oder Propanol oder Säuren, wie Essigsäure.

Die erfindungsgemäßen Verfahren (d) und (k) werden gegebenenfalls in Gegenwart eines geeigneten Reaktionshilfsmittels durchgeführt. Als solche kommen insbesondere anorganische Mineralsäuren wie beispielsweise Salzsäure oder Schwefelsäure in Frage. Es ist auch möglich, die als Ausgangsstoffe in Frage kommenden Phenylhydrazine der Formel (VII) in Form von entsprechenden Säureadditionssalzen, wie beispielsweise Hydrochloriden einzusetzen.

Die Reaktionstemperaturen können bei der Durchführung der erfindungsgemäßen Verfahren (d) und (k) in einem größeren Bereich variiert werden. Im allgemeinen arbeitet man bei Temperaturen zwischen -20 °C und 180 °C, vorzugsweise bei Temperaturen zwischen 20 °C und 120 °C.

Zur Durchführung der erfindungsgemäßen Verfahren (d) und (k) setzt man pro Mol an Phenylhydrazin der Formel (II) bzw. an einem entsprechenden Säureadditionssalz im allgemeinen 0.5 bis 10 Mol an 1,3-Diketon der Formel (VIII) oder an einem entsprechenden Derivat bzw. Hydroxyisobenzfuranon der Formel (XIV) und gegebenenfalls 0.01 bis 1 Mol an Reaktionshilfsmittel ein. Die Reaktionsdurchführung, Aufarbeitung und Isolierung der N-Aryl-Stickstoffheterocyclen der Formel (Id) bzw. (Il) erfolgt nach allgemein üblichen Methoden (vgl. auch die Herstellungsbeispiele).

Bei Verwendung von 1,3-Diketonen der Formel (VIII), bei welchen der Substituent $R^{10}$ verschieden von dem Substituenten $R^{11-1}$ ist, erhält man in der Regel Isomerengemische aus Verbindungen der Formel (Id-1),

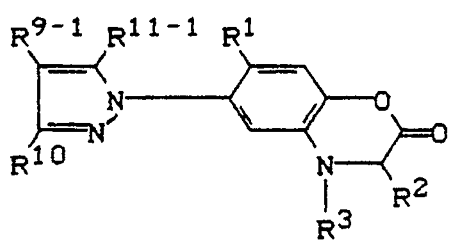

(Id-1)

und Verbindungen der Formel (Id-2)

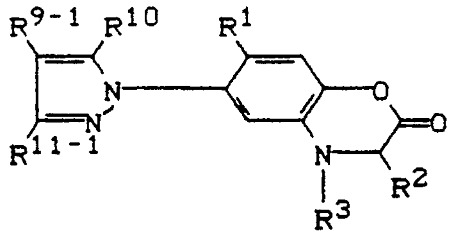

(Id-2)

Aus diesen Isomerengemischen lassen sich mit üblichen Trennverfahren (Destillation, Kristallisation, Chromatographie) die gewünschten Reaktionsprdukte der Formel (Id) isolieren.

Als Halogenierungsmittel zur Durchführung des erfindungsgemäßen Verfahrens (e) kommen übliche Halogenierungsmittel infrage. Mit besonderem Vorzug verwendet man Sulfurylchlorid, elementares Chlor oder elementares Brom als Halogenierungsmittel.

Als Verdünnungsmittel zur Durchführung des erfindungsgemäßen Verfahrens (e) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, O-Dichlorbenzol, Petrolether, Hexan, Cyclohexan, Dichlormethan, Chloroform oder Tetrachlorkohlenstoff.

Die Reaktionstemperaturen können bei der Durchführung des erfindungsgemäßen Verfahrens (e) in einem größeren Bereich variiert werden. Im allgemeinen arbeitet man bei Temperaturen zwischen 0 °C und 150 °C, vorzugsweise bei Temperaturen zwischen 35 °C und 70 °C.

Zur Durchführung des erfindungsgemäßen Verfahrens (e) setzt man pro Mol an N-Aryl-Stickstoffheterocyclus der Formel (Ie) im allgemeinen 1 bis 5 Mol, vorzugsweise 1 bis 2 Mol an Halogenierungsmittel ein.

Die Reaktionsdurchführung, Aufarbeitung und Isolierung der Reaktionsprodukte erfolgt nach allgemein üblichen Methoden (vgl. auch die Herstellungsbeispiele).

Als Halogenierungsmittel zur Durchführung des erfindungsgemäßen Verfahrens (f) kommen ebenfalls übliche Halogenierungsmittel infrage. Mit besonderem Vorzug verwendet man anorganische Säurehalogenide wie beispielsweise Phosphoroxychlorid, Thionylchlorid, Phosgen, Phosphortribromid oder Diphosgen ($Cl_3C$-O-CO-Cl).

Als Verdünnungsmittel zur Durchführung des erfindungsgemäßen Verfahrens (f) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, O-Dichlorbenzol, Petrolether, Hexan, Cyclohexan, Dichlormethan, Chloroform oder Tetrachlorkohlenstoff oder basische Verdünnungsmittel wie beispielsweise Pyridin. Es ist auch möglich, einen entsprechenden Überschuß an Halogenierungsmittel gleichzeitig als Verdünnungsmittel einzusetzen.

Das erfindungsgemäße Verfahren (f) wird vorzugsweise in Gegenwart eines geeigneten Reaktionshilfsmittels durchgeführt. Als solche kommen insbesondere übliche Hilfsnukleophile wie beispielsweise Triphenylphosphin, Dimethylanilin oder Dimethylformamid infrage.

Die Reaktionstemperaturen können bei der Durchführung des erfindungsgemäßen Verfahrens (f) in einem größeren Bereich variiert werden. Im allgemeinen arbeitet man bei Temperaturen zwischen 0 °C und 200 °C, vorzugsweise bei Temperaturen zwischen 30 °C und 150 °C.

Zur Durchführung des erfindungsgemäßen Verfahrens (f) setzt man pro Mol an N-Arylpyrazolinon der Formel (IX) im allgemeinen 1 bis 10 Mol, vorzugsweise 1 bis 5 Mol an Halogenierungsmittel und gegebenenfalls 0.01 bis 1 Mol, vorzugsweise 0.05 bis 0.1 Mol an Reaktionshilfsmittel ein.

Die Reaktionsdurchführung, Aufarbeitung und Isolierung der Reaktionsprodukte erfolgt nach allgemein üblichen Methoden (vgl. auch die Herstellungsbeispiele).

Als Verdünnungsmittel zur Durchführung der erfindungsgemäßen Verfahren (g) und (j) kommen inerte organische Losungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, O-Dichlorbenzol, Petrolether, Hexan, Cyclohexan, Dichlormethan, Chloroform oder Tetrachlorkohlenstoff.

Die erfindungsgemäßen Verfahren (g) und (j) werden vorzugsweise in Gegenwart eines geeigneten Reaktionshilfsmittels durchgeführt. Als solche kommen alle üblichen Basen in Frage. Mit besonderem Vorzug verwendet man tertiäre Amine, wie Triethylamin, N,N- Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung der erfindungsgemäßen Verfahren (g) und (j) in einem größeren Bereich variiert werden. Im allgemeinen arbeitet man bei Temperaturen zwischen -20 °C und 180 °C, vorzugsweise bei Temperaturen zwischen 0 °C und 150 °C.

Zur Durchführung des erfindungsgemäßen Verfahrens (j) setzt man pro Mol an Amidrazon der Formel (XIII) im allgemeinen 1 bis 5 Mol, vorzugsweise 1 bis 1,5 Mol an Phosgen und gegebenenfalls 1 bis 5 Mol, vorzugsweise 1 bis 3 Mol an Reaktionshilfsmittel ein.

Die Reaktionsdurchführung, Aufarbeitung und Isolierung der Reaktionsprodukte erfolgt nach allgemein üblichen Methoden.

Zur Durchführung des erfindungsgemäßen Verfahrens (g) setzt man pro Mol an Phenylhydrazid der Formel (X) im allgemeinen 1 bis 5 Mol, vorzugsweise 1 bis 1,5 Mol an Phosgen und gegebenenfalls 1 bis 5 Mol, vorzugsweise 1.0 bis 3.0 Mol an Reaktionshilfsmittel ein.

Die Reaktionsdurchführung, Aufarbeitung und Isolierung der Reaktionsprodukte erfolgt nach allgemein üblichen Methoden (vgl. auch die Herstellungsbeispiele).

Als Verdünnungsmittel zur Durchführung des erfindungsgemäßen Verfahrens (h) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyloder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester, Sulfoxide, wie Dimethylsulfoxid oder Alkohole, wie Methanol, Ethanol oder Propanol.

Die Reaktionstemperaturen können bei der Durchführung des erfindungsgemäßen Verfahrens (h) in einem größeren Bereich variiert werden. Im allgemeinen arbeitet man bei Temperaturen zwischen 0 °C und 180 °C, vorzugsweise bei Temperaturen zwischen 50 °C und 150 °C.

Zur Durchführung des erfindungsgemäßen Verfahrens (h) setzt man pro Mol an Phenylhydrazin der Formel (VII) im allgemeinen 1 bis 3 Mol, vorzugsweise 1 bis 2 Mol an Iminocarbonester der Formel (XI) ein.

Die Reaktionsdurchführung, Aufarbeitung und Isolierung der Reaktionsprodukte erfolgt nach allgemein üblichen Methoden (vgl. auch die Herstellungsbeispiele).

Zur Durchführung des erfindungsgemäßen Verfahrens (i) kommen als Verdünnungsmittel inerte organische Lösungsmittel infrage. Vorzugsweise verwendet man aliphatische, cyclische oder aromatische, gegebenenfalls halogenierte Kohlenwasserstoffe, wie beispielsweise Benzin, Benzol, Toluol, Xylol, Pentan, Hexan, Heptan, Cyclohexan, Petrolether, Ligroin, Methylenchlorid, Chloroform, Tetrachlorkohlenstoff, Chlorbenzol oder Dichlorbenzol, Ether, wie Diethylether, Diisopropylether, Dioxan, Tetrahydrofuran oder Ethylenglykoldiethylether oder -dimethylether, Ketone, wie Aceton, Butanon, Methylisopropylketon oder Methylisobutylketon, Ester, wie Essigsäureethylester, Nitrile, wie Acetonitril oder Propionitril oder Amide, wie Dimethylformamid, Dimethylacetamid, N-Methylpyrrolidon oder Hexamethylphosphorsäuretriamid. Verwendet man Alkylierungsmittel der Formel (XII) in flüssiger Form, so ist es auch möglich, diese in entsprechendem Überschuß gleichzeitig als Verdünnungsmittel einzusetzen.

Als Reaktionshilfsmittel zur Durchführung des erfindungsgemäßen Verfahrens (i) kommen alle üblicherweise verwendbaren anorganischen und organischen Basen in Frage. Vorzugsweise verwendet man Alkalimetallhydride, -hydroxide, -amide, -carbonate oder -hydrogencarbonate, wie beispielsweise Natriumhydrid, Natriumamid, Natriumhydroxid, Natriumcarbonat, Kaliumcarbonat oder Natriumhydrogencarbonat, oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung des Herstellungsverfahrens (i) in einem größeren Bereich variiert werden. Im allgemeinen arbeitet man zwischen -20 °C und +150 °C, vorzugsweise zwischen 0 °C und +100 °C.

Zur Durchführung des erfindungsgemäßen Verfahrens (i) setzt man pro Mol an N-Aryl-Stickstoffheterocyclus der Formel (Ii) im allgemeinen 1 bis 15 Mol, vorzugsweise 1 bis 5 Mol an Alkylierungsmittel der Formel (XII) und gegebenenfalls 1 bis 3 Mol, vorzugsweise 1 bis 2 Mol an Reaktionshilfsmittel ein.

Die Reaktionsdurchführung, Aufarbeitung und Isolierung der Reaktionsprodukte erfolgt nach allgemein üblichen Methoden (vgl. auch die Herstellungsbeispiele).

Zur Durchführung des erfindungsgemäßen Verfahrens (l) kommen als Verdünnungsmittel inerte organische Lösungsmittel infrage. Vorzugsweise verwendet man aliphatische, alicyclische oder aromatische, gegebenenfalls halogenierte Kohlenwasserstoffe, wie beispielsweise Benzin, Benzol, Toluol, Xylol, Pentan, Hexan, Heptan, Cyclohexan, Petrolether, Ligroin, Methylenchlorid, Chloroform, Tetrachlorkohlenstoff, Chlorbenzol oder Dichlorbenzol, Ether, wie Diethylether, Diisopropylether, Dioxan, Tetrahydrofuran oder Ethylenglykoldiethylether oder -dimethylether, Ketone, wie Aceton, Butanon, Methylisopropylketon oder Methylisobutylketon, Ester, wie Essigsäureethylester, Säuren, wie Essigsäure, Nitrile, wie Acetonitril oder Propionitril, Amide, wie Dimethylformamid, Dimethylacetamid, N-Methylpyrrolidon oder Hexamethylphosphorsäuretriamid. Verwendet man als Reaktionspartner Alkylierungsmittel der Formel (XVI) in flüssiger Form, so ist es

auch möglich, diese in entsprechendem Überschuß gleichzeitig als Verdünnungsmittel einzusetzen.

Als Reaktionshilfsmittel zur Durchführung des erfindungsgemäßen Verfahrens (I) kommen alle üblicherweise verwendbaren anorganischen und organischen Basen in Frage. Vorzugsweise verwendet man Alkalimetallhydride, -hydroxide, -amide, -carbonate oder -hydrogencarbonate, wie beispielsweise Natriumhydrid, Natriumamid, Natriumhydroxid, Natriumcarbonat oder Natriumhydrogencarbonat, oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (I) in einem größeren Bereich variiert werden. Im allgemeinen arbeitet man zwischen -20 °C und +150 °C, vorzugsweise zwischen 0 °C und +100 °C.

Zur Durchführung des erfindungsgemäßen Verfahrens (I) setzt man pro Mol N-Aryl-Stickstoffheterocyclus der Formel (In) im allgemeinen 1 bis 20 Mol, vorzugsweise 1 bis 15 Mol, an Alkylierungs- bzw. Acylierungsmittel der Formel (XVI) und gegebenenfalls 1 bis 3 Mol, vorzugsweise 1 bis 2 Mol an Reaktionshilfsmittel, ein. Die Reaktionsdurchführung, Aufarbeitung und Isolierung der Reaktionsprodukte der Formel (Im) erfolgt nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera. Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Dle Verbindungen eignen sich in Abhängigkeit von der Konzentration zur Totalunkrautbekämpfung z.B. auf Industrie- und Gleisanlagen und auf Wogen und Plätzen mit und ohne Baumbewuchs. Ebenso können die Verbin dungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekampfung in einjährigen Kulturen eingesetzt werden.

Die erfindungsgemäßen Wirkstoffe der Formel (I) eignen sich insbesondere zur selektiven Unkrautbekämpfung von mono- und dikotylen Unkräutern in mono- und dikotylen Kulturen im Vor- und Nachauflaufverfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslö sungsmittel verwendet werden. Als flüssige Losungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier-und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B.
Aryloxalkansäuren wie 2,4 D, 2,4 DP, 2,4 DB, MCPA, MCPP, Fluroxypyr; Aryloxy-phenoxy-alkansäureester wie Diclofop-methyl, Fenoxaprop, Fluazifop-butyl, Quizalofop, Haloxyfop; Arylcarbonsäuren wie Clopyralid; Azinone wie Chloridazon, Norflurazon; Carbamate wie Phenmedipham; Chloracetanilide wie Alachlor, Metazachlor, Metolachlor; Dinitroaniline wie Oryzalin, Pendimethalin, Trifluralin; Diphenylether wie Acifluorfen, Bifenox, Fomesafen, Lactofen, Oxyfluorfen; Harnstoffe wie Chlortoluron, Flurmeturon, Isoproturon, Methabenzthiazuron; Hydroxylamine wie Alloxydim, Sethoxydim; Imidazolinone wie Imazethapyr, Imazamethabenz, Imazaquin; Nitrile wie Bromoxynil, Ioxynil; Oxyacetamide wie Mefenacet; Sulfonylharnstoffe wie Bensulfuron, Chlorimuron, Chlorsulfuron, Metsulfuron, Thiameturon; Thiolcarbamate wie Cycloate, EPTC, Molinate, Triallate; Triazine wie Atrazin, Cyanazin, Simazin, Simetryne, Terbutryne; Triazinone wie Ethiozin, Hexazinon, Metamitron, Metribuzin oder Bentazone, Cinmethylin, Fluridone, Pyridate oder Dimethazone in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,001 und 10 kg Wirkstoff pro Hektar Bodenfläche, vorzugsweise zwischen 0,005 und 5 kg pro ha.

Herstellungsbeispiele

Beispiel 1

2-[7-fluor-3H-1,4-benzoxazin-2(4H)-on-6-yl]-4,5,6,7-tetrahydro-2H-isoindol-1,3-dion

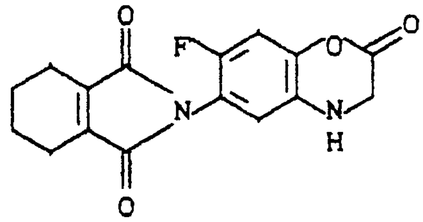

Verfahren (a)

Eine Mischung von 5 g (0,027 mol) 6-Amino-7-fluor-3H-1,4-benzoxazin-2(4H)-on, 4,2 g (0,027 mol) 3,4,5,6-Tetrahydrophthalsäureanhydrid und 15 ml Eisessig wird 4h bei Rückfluß gerührt. Die Reaktionsmischung wird in Eiswasser eingetragen und der ausgefallene Feststoff abgesaugt. Nach Waschen und Trocknen werden 6,5 g (76 % der Theorie) des oben genannten Produktes erhalten, Schmelzpunkt: 228-233 °C.

Vorprodukte:

Beispiel III-1

6-Amino-7-fluor-3H-1,4-benzoxazin-2(4H)-on

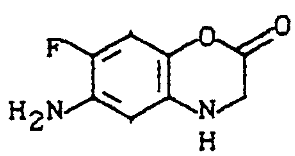

Zu einer Suspension von 22 g (0,39 mol) Eisenpulver in 170 ml 5 proz. Essigsäure werden bei 60 °C portionsweise 22 g (0,1 mol) 6-Nitro-7-fluor-3H-1,4-benzoxazin-2(4H)-on eingetragen; dabei erwärmt sich die Suspension auf 90 °C. Man läßt noch weitere 30 min bei 90 °C rühren, und filtriert nach dem Abkühlen vom Ungelösten ab. Der Filtrationsrückstand wird mehrmals mit warmem Methanol aufgeschlämmt und filtriert. Die vereinigten Methanolfiltrate ergeben nach dem Einengen 13 g (71 % der Theorie) 6-Amino-7-fluor-3H-1,4-benzoxazin-2(4H)-on (Fp. >250 °C).

Beispiel XVII-1

6-Nitro-7-fluor-3H-1,4-benzoxazin-2(4H)-on

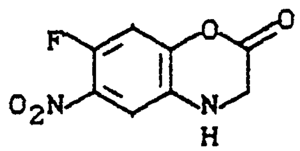

Zu einer Lösung von 5 g (0,03 mol) 7-Fluor-3H-1,4-benzoxazin-2(4H)-on in 50 ml konzentrierter Schwefelsäure werden bei -5°C bis 0°C 4,6 ml 60 proz. Salpetersäure getropft. Nach 1,5 stündigem Nachrühren bei 0°C gibt man auf Eis, saugt ab und wäscht den Rückstand mit Wasser.

Nach Trocknen werden 4,8 g (75 % der Theorie) 6-Nitro-7-fluor-3H-1,4-benzoxazin-2(4H)-on mit dem Schmelzpunkt von 196-202°C erhalten.

Beispiel XX-1

7-Fluor-3H-1,4-benzoxazin-2(4H)-on

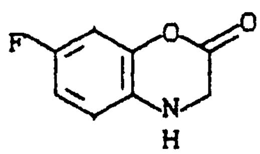

Zu einer Suspension von 50 g (0,89 mol) Eisenpulver in 450 ml 5 proz. wässriger Essigsäure werden bei 60°C portionsweise 50 g (0,17 mol) Methylsulfonyloxyessigsäure-5-fluor-2-nitro-phenylester eingetragen. Dabei erwärmt sich die Suspension auf 90°C. Man läßt noch weitere 30 min bei 90°C rühren und filtriert nach dem Abkühlen vom Ungelösten ab. Der Filtrationsrückstand wird mehrmals mit warmem Methanol aufgeschlämmt und filtriert. Die vereinigten Methanolfiltrate ergeben nach dem Einengen 21,3 g (75 % der Theorie) 7-Fluor-3H-1,4-benzoxazin-2(4H)-on mit dem Schmelzpunkt 199°C.

Beispiel XIX-1

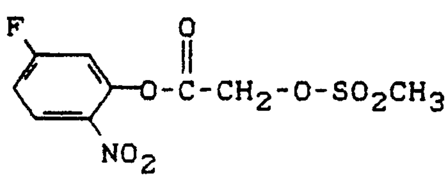

Methylsulfonyloxyessigsäure-5-fluor-2-nitro-phenylester

Zu einer Lösung von 92 g (0,40 mol) 5-Fluor-2-nitrophenol und 81 ml (0,59 mol) Triethylamin in 250 ml Toluol werden über einen Zeitraum von 30 min eine Lösung von 92 g (0,53 mol) Methylsulfonyloxyessigsäurechlorid in 250 ml Toluol zugetropft. Man läßt über Nacht bei Raumtemperatur rühren, und engt nach dem Abfiltrieren des Triethylaminhydrochlorids zur Trockne ein.

Man erhält 80,4 g (69 % der Theorie) Methylsulfonyloxyessigsäure-5-fluor-2-nitro-phenylester als

wachsartigen Feststoff, der ohne weitere Reinigung umgesetzt werden kann.

IR ($CH_2Cl_2$): 180, 1610, 1540 $cm^{-1}$.

Beispiel 2

2-[7-Fluor-4-propargyl-3H-1,4-benzoxazin-2(4H)-on-6-yl]-4,5,6,7-tetrahydro-2H-isoindol-1,3-dion

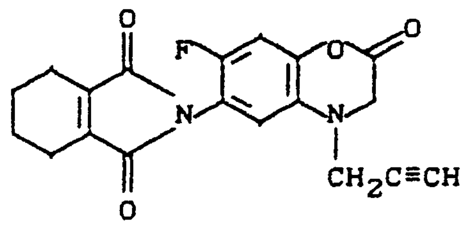

Verfahren (I)

Zu einer Suspension von 0,42 g (0,014 mol, 80 % in Öl) NaH in 5 ml Tetrahydrofuran tropft man bei 0 °C eine Lösung von 3 g (0,0095 mol) 2-[7-Fluor-3H-1,4-benzoxazin-2(4H)-on-6-yl]-4,5,6,7-tetrahydro-2H-isoindol-1,3-dion in 5 ml Tetrahydrofuran. Nach 30 min Rühren bei Raumtemperatur werden 1,6 ml (0,014 mol, 80 % in Toluol) Propargylbromid zugetropft und 2h bei Rückfluß gerührt. Nach wäßriger Aufarbeitung erhält man 2,5 g (74 % der Theorie) 2-[7-Fluor-4-propargyloxy-3H-1,4-benzoxazin-2(4H)-on-6-yl]-4,5,6,7-tetrahydro-2H-isoindol-1,3-dion mit dem Schmelzpunkt 189-190 °C.

In entsprechender Weise und gemäß den allgemeinen Angaben zur Herstellung erhält man die folgenden N-Aryl-Stickstoffheterocyclen der Formel (I)

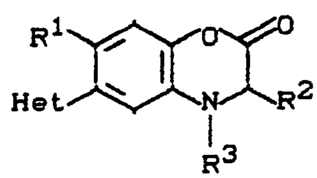

**Tabelle 1**

| Bsp. Nr. | $R^1$ | $R^2$ | $R^3$ | Het | Schmelzpunkt/°C |
|---|---|---|---|---|---|
| 3 | F | H | $-CH_2CN$ | 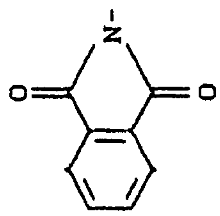 | 217-220 |
| 4 | F | H | $-COCH_3$ | " | 155-163 |
| 5 | F | H | $-CO_2CH_2CH_2OCH_3$ | " | 220 |
| 6 | F | H | $-SO_2CH_3$ | " | 203-208 |
| 7 | F | H | $-CH_2-C{\equiv}CH$ | 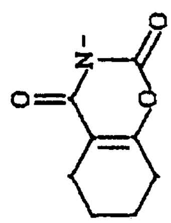 | 210,5-212 |

Beispiel 8

3-Chlor-2-[7-fluor-4-propargyl-3H-1,4-benzoxazin-2(4H)-on-6-yl]-4,5,6,7-tetrahydro-2H-indazol

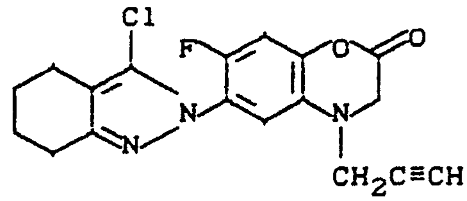

Verfahren (f)

Eine Mischung von 3 g (8,8 mmol) 2-(7-Fluor-4-propargyl-3H-1,4-benzoxazin-2(4H)-on-6-yl)-2,3,4,5,6,7-hexahydro2H-indazol-3-on und 2,5 ml (27 mmol) Phosphoroxychlorid wird 3h auf Rückfluß erhitzt. Nach dem Abkühlen nimmt man den Rückstand in Methylenchlorid auf, wäscht nacheinander mit Wasser, 5 proz. Natronlauge und zuletzt mit Wasser, trocknet und engt ein. Der Rückstand ergibt nach säulenchromatografischer Reinigung (Kieselgel, Petrolether-Essigsäureethylester = 2:1) 0,8 g (25 % d.Th.) 3-Chlor-2-[7-fluor-4-propargyl-3H-1,4-benzoxazin-2(4H)-on-6-yl]-4,5,6,7-tetrahydro-2H-indazol.

Massenspektrum: m/z (% rel. Int.): 359 (80).

Vorprodukte:

Beispiel IX-1

2-(7-Fluor-4-propargyl-3H-1,4-benzoxazin-2(4H)-on-6-yl)-2,3,4,5,6,7-hexahydro-2H-indazol-3-on

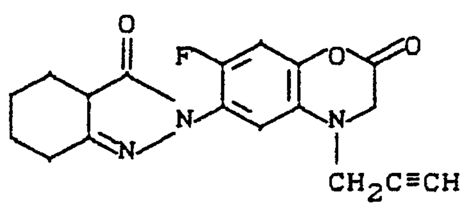

Eine Mischung aus 5 g (0,021 mol) 7-Fluor-4-propargyl-3H-1,4-benzoxazin-2(4H)-on-6-ylhydrazin und 3,6 g (0,021 mol) Cyclohexanon-2-carbonsäureethylester werden in ml Ethanol und 0,3 ml konz. Salzsäure 6 h auf Rückfluß erhitzt.

Man läßt abkühlen, gibt auf Eis und extrahiert die wäßrige Phase mehrmals mit Methylenchlorid. Die vereinigten organischen Phasen werden getrocknet und eingeengt. Man erhält so 5,1 g (71 % der Theorie) 2-(7-Fluor-4-propargyl-3H-1,4-benzoxazin-2(4H)-on-6-yl)-2,3,4,5,6,7-hexahydro-2H-indazol-3-on.

Massenspektrum: m/z (% rel. Int): 341 (100), 312 (10), 246 (15), 204 (30).

Beispiel VII-1

7-Fluor-4-propargyl-3H-1,4-benzoxazin-2-(4H)-on-6-ylhydrazin

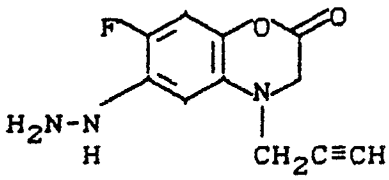

In einer Lösung von 6 g (0,027 mol) 6-Amino-7-fluor-3H-1,4-benzoxazin-2-(4H)-on in 30 ml konz. Salzsäure tropft man bei 0 °C eine gesättigte, wäßrige Lösung von 2,4 g (0,035 mol) Natriumnitrit und rührt noch 2 h bei 0 °C nach. Die Reaktionsmischung wird auf -30 °C gekühlt und eine Lösung bestehend aus 13 g (0,069) Zinn(II)chlorid in 12 ml konz. Salzsäure zugetropft. Man rührt 8 h bei 0 °C nach, gibt auf Eis und stellt mit 6N-Natronlauge einen pH-Wert von 10 ein. Nach Absaugen und Trocknen werden 6 g (94,5 % der Theorie) 7-Fluor-4-propargyl-3H-1,4-benzoxazin-2(4H)-on-6-ylhydrazin mit dem Schmelzpunkt von 146 °C erhalten.

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle) 100 % = totale Vernichtung

In diesem Test zeigen beispielsweise die Verbindungen gemäß Beispiel-Nr. 1, 2, 3, 4, 5 und 7 bei guter Nutzpflanzenverträglichkeit eine sehr gute herbizide Wirkung.

Beispiel B

Pre-emergence-Test

Lösungsmittel: 5 Gewichtsteile Aceton Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

In diesem Test zeigen beispielsweise die Verbindungen gemäß Beispiel-Nr. 1, 2, 3, 4 und 5 eine sehr gute Nutzpflanzenverträglichkeit in mono- und dikotylen Kulturen und eine sehr gute herbizide Wirkung.

## Ansprüche

1. N-Aryl-Stickstoffheterocyclen der allgemeinen Formel (I),

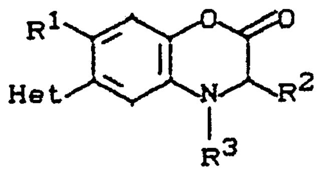

(I)

in welcher
Het für einen Heterocyclus der Formel

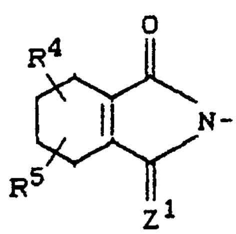
;
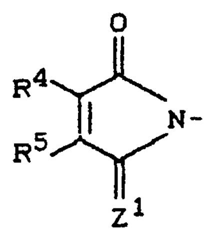
;
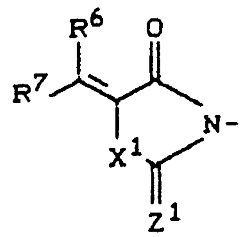
;

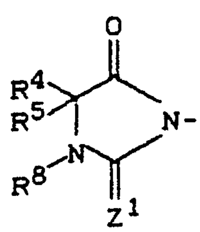
;
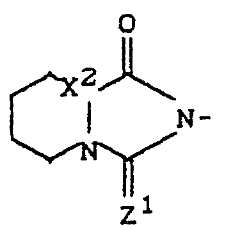
;
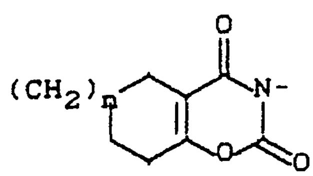
;

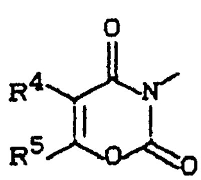
;
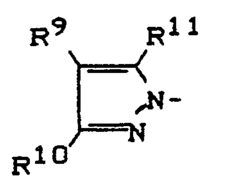
;
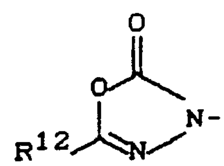
;

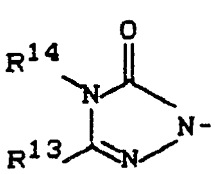
oder
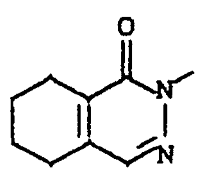
steht,

$R^1$ für Wasserstoff oder Halogen steht,
$R^2$ für Wasserstoff oder Alkyl steht,
$R^3$ für Wasserstoff oder jeweils gegebenenfalls substituiertes Alkyl, Alkenyl, Alkinyl, Cycloalkyl, Cycloalkenyl, Alkylsulfonyl, Arylsulfonyl, Alkylcarbonyl oder Alkoxycarbonyl steht,
$R^4$ und $R^5$ unabhängig voneinander jeweils für Wasserstoff oder Alkyl stehen,
$R^6$ und $R^7$ entweder unabhängig voneinander jeweils für Wasserstoff oder Alkyl stehen oder gemeinsam für einen zweifach verknüpften Alkandiylrest stehen,
$R^8$ für Wasserstoff, Alkyl oder gegebenenfalls substituiertes Aryl steht,
$R^9$ für Wasserstoff, Halogen, Alkyl oder Halogenalkyl steht,

$R^{10}$ für Wasserstoff, Alkyl oder Halogenalkyl steht oder
$R^9$ und $R^{10}$ gemeinsam für zweifach verknüpftes Alkandiyl stehen,
$R^{11}$ für Wasserstoff, Halogen, Alkyl oder Halogenalkyl steht,
$R^{12}$ und $R^{13}$ unabhängig voneinander für Wasserstoff, Alkyl, Alkoxyalkyl, Halogenalkyl, Alkenyl, Halogenalkenyl, Alkinyl, Halogenalkinyl oder für gegebenenfalls substituiertes Cycloalkyl stehen,
$R^{14}$ für Wasserstoff, Alkyl, Halogenalkyl, Alkenyl, Halogenalkenyl, Alkinyl oder Halogenalkinyl steht oder
$R^{13}$ und $R^{14}$ gemeinsam für zweifach verknüpftes Alkandiyl stehen,
$X^1$ für Sauerstoff, eine $-CH_2$-Gruppe oder eine

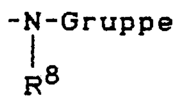

steht,
$X^2$ für Stickstoff oder eine CH-Gruppe steht,
$Z^1$ für Sauerstoff oder Schwefel steht und
n für die Zahl 0 oder 1 steht,
2. N-Aryl-Stickstoffheterocyclen der Formel (I) gemäß Anspruch 1, in welcher
Het für einen Heterocyclus der Formel steht

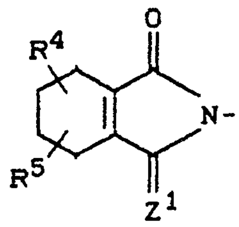 ; 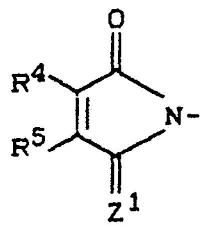 ; 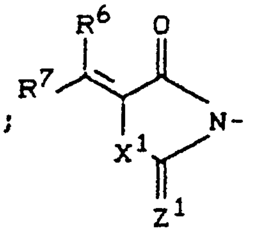 ;

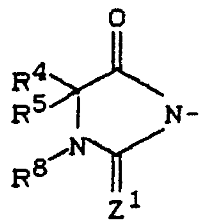 ; 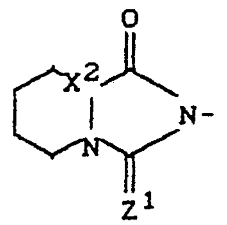 ; 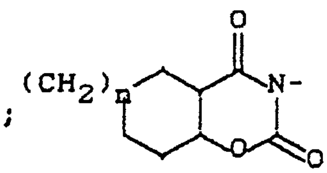 ;

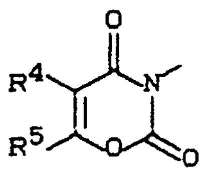 ; 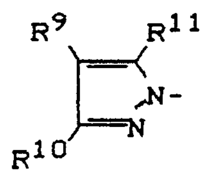 ; 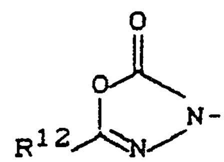 ;

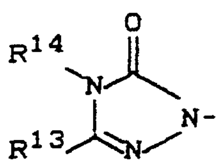 oder 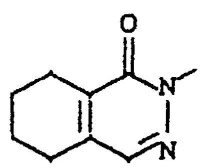

steht,
$R^1$ für Wasserstoff oder Halogen steht,
$R^2$ für Wasserstoff oder Alkyl mit 1 bis 4 Kohlenstoffatomen steht,
$R^3$ für Wasserstoff, Alkyl mit 1 bis 8 Kohlenstoffatomen, Alkenyl mit 2 bis 8 Kohlenstoffatomen, Alkinyl mit 3 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 3 bis 8 Kohlenstoffatomen und 1 bis 13 gleichen oder verschiedenen Halogenatomen, Alkylsulfonyl, Cyanalkyl, Alkoxyalkyl, Alkylthioalkyl, Halogenalkoxyalkyl, Alkoxyalkoxyalkyl, Bis-(Alkoxy)alkyl, Bis-(Alkylthio)alkyl, Alkylcarbonyl, Alkoxycarbonyl, Alkylcarbonylalkyl, Alkoxycarbonylalkyl oder Alkoxyalkoxycarbonylalkyl mit jeweils 1 bis 8 Kohlenstoffatomen in den einzelnen Alkylteilen und gegebenenfalls 1 bis 9 gleichen oder verschiedenen Halogenatomen oder für jeweils gegebenenfalls einfach oder mehrfach, gleich oder verschieden substituiertes Cycloalkyl, Ccyloalkenyl, Cycloalkyloxycarbonylalkyl oder Cycloalkylalkyl mit jeweils 3 bis 7 Kohlenstoffatomen im Cycloalkylteil und gegebenenfalls 1 bis 4 Kohlenstoffatomen im Alkylteil steht, wobei als Substituenten im Cycloalkylteil jeweils in Frage kommen:
Halogen sowie Alkyl oder Alkoxy mit jeweils 1 bis 4 Kohlenstoffatomen, $R^3$ außerdem für jeweils gegebenenfalls durch Alkyl mit 1 bis 4 Kohlenstoffatomen substituiertes Oxetanyl alkyl, Tetrahydrofuranylalkyl oder Tetrahydropyranylalkyl mit jeweils 1 bis 3 Kohlenstoffatomen in den jeweiligen Alkylteilen steht und für jeweils gegebenenfalls einfach oder mehrfach gleich oder verschieden substituiertes Aralkyl mit 6 bis 10 Kohlenstoffatomen im Arylteil und 1 bis 4 Kohlenstoffatomen im Alkylteil oder für Arylsulfonyl mit 6 bis 10

Kohlenstoffatomen steht, wobei als Arylsubstituenten jeweils in Frage kommen: Halogen, Cyano, Nitro, Alkyl, Alkoxy, Alkylthio oder Alkoxycarbonyl mit jeweils 1 bis 4 Kohlenstoffatomen oder Halogenalkyl, Halogenalkoxy oder Halogenalkylthio mit jeweils 1 bis 4 Kohlenstoffatomen und 1 bis 9 gleichen oder verschiedenen Halogenatomen,

$R^4$ und fis unabhängig voneinander jeweils für Wasserstoff oder Alkyl mit 1 bis 4 Kohlenstoffatomen stehen,

$R^6$ und $R^7$ entweder unabhängig voneinander jeweils für Wasserstoff oder für Alkyl mit 1 bis 4 Kohlenstoffatomen stehen oder gemeinsam für einen zweifachverknüpften Alkandiylrest mit 2 bis 7 Kohlenstoffatomen stehen

$R^8$ für Wasserstoff, Alkyl mit 1 bis 4 Kohlenstoffatomen oder für gegebenenfalls einfach oder mehrfach, gleich oder verschieden substituiertes Phenyl steht, wobei als Substituenten in Frage kommen: Halogen, Cyano, Nitro, Alkyl, Alkoxy oder Alkylthio mit jeweils 1 bis 4 Kohlenstoffatomen sowie Halogenalkyl, Halogenalkoxy oder Halogenalkylthio mit jeweils 1 bis 4 Kohlenstoffatomen und 1 bis 9 gleichen oder verschiedenen Halogenatomen und

$R^9$ für Wasserstoff, Fluor, Chlor, Brom, Iod, Alkyl mit 1 bis 6 Kohlenstoffatomen oder für Halogenalkyl mit 1 bis 4 Kohlenstoffatomen und 1 bis 9 gleichen oder verschiedenen Halogenatomen steht oder

$R^{10}$ für Wasserstoff, Alkyl mit 1 bis 6 Kohlenstoffatomen oder Halogenalkyl mit 1 bis 4 Kohlenstoffatomen und 1 bis 9 gleichen oder verschiedenen Halogenatomen steht oder

$R^9$ und $R^{10}$ gemeinsam für einen zweifach verknüpften Alkandiylrest mit 2 bis 6 Kohlenstoffatomen stehen,

$R^{11}$ für Wasserstoff, Fluor, Chlor, Brom, Iod, Alkyl mit 1 bis 6 Kohlenstoffatomen oder Halogenalkyl mit 1 bis 4 Kohlenstoffatomen und 1 bis 9 gleichen oder verschiedenen Halogenatomen steht,

$R^{12}$ für Wasserstoff, Alkyl mit 1 bis 6 Kohlenstoffatomen, Alkenyl mit 3 bis 6 Kohlenstoffatomen, Alkinyl mit 3 bis 6 Kohlenstoffatomen, Halogenalkyl mit 1 bis 4 Kohlenstoffatomen und 1 bis 9 gleichen oder verschiedenen Halogenatomen, Halogenalkenyl mit 3 bis 6 Kohlenstoffatomen und 1 bis 5 gleichen oder verschiedenen Halogenatomen, Halogenalkinyl mit 3 bis 6 Kohlenstoffatomen und 1 bis 5 gleichen oder verschiedenen Halogenatomen oder Alkoxyalkyl mit jeweils 1 bis 4 Kohlenstoffatomen in den einzelnen Alkylteilen steht oder für gegebenenfalls einfach oder mehrfach, gleich oder verschieden substituiertes Cycloalkyl mit 3 bis 7 Kohlenstoffatomen steht, wobei als Substituenten infrage kommen: Halogen, Alkyl oder Alkoxy mit jeweils 1 bis 4 Kohlenstoffatomen,

$R^{13}$ für Wasserstoff, Alkyl mit 1 bis 6 Kohlenstoffatomen, Alkenyl mit 3 bis 6 Kohlenstoffatomen, Alkinyl mit 3 bis 6 Kohlenstoffatomen, Halogenalkyl mit 1 bis 4 Kohlenstoffatomen und 1 bis 9 gleichen oder verschiedenen Halogenatomen, Halogenalkenyl mit 3 bis 6 Kohlenstoffatomen und 1 bis 5 gleichen oder verschiedenen Halogenatomen, Halogenalkinyl mit 3 bis 6 Kohlenstoffatomen und 1 bis 5 gleichen oder verschiedenen Halogenatomen oder Alkoxyalkyl mit jeweils 1 bis 4 Kohlenstoffatomen in den einzelnen Alkylteilen steht oder für gegebenenfalls einfach oder mehrfach, gleich oder verschieden substituiertes Cycloalkyl mit 3 bis 7 Kohlenstoffatomen steht, wobei als Substituenten in Frage kommen: Halogen, Alkyl oder Alkoxy mit jeweils 1 bis 4 Kohlenstoffatomen und

$R^{14}$ für Wasserstoff, Alkyl mit 1 bis 6 Kohlenstoffatomen, Alkenyl mit 3 bis 6 Kohlenstoffatomen, Alkinyl mit 3 bis 6 Kohlenstoffatomen, Halogenalkyl mit 1 bis 4 Kohlenstoffatomen und 1 bis 9 gleichen oder verschiedenen Halogenatomen, Halogenalkenyl mit 3 bis 6 Kohlenstoffatomen und 1 bis 5 gleichen oder verschiedenen Halogenatomen oder Halogenalkinyl mit 3 bis 6 Kohlenstoffatomen und 1 bis 5 gleichen oder verschiedenen Halogenatomen steht oder

$R^{13}$ und $R^{14}$ gemeinsam für einen zweifach verknüpften Alkandiylrest mit 2 bis 6 Kohlenstoffatomen stehen

$X^1$ für Sauerstoff, eine $CH_2$-Gruppe oder eine

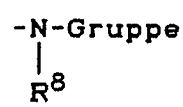

steht,

$X^2$ für Stickstoff oder eine CH-Gruppe steht,

$Z^1$ für Sauerstoff oder Schwefel steht und

n für die Zahl 0 oder 1 steht.

3. N-Aryl-Stickstoffheterocyclen der Formel (I) gemäß Anspruch 1, in welcher

Het für einen Heterocyclus der Formel

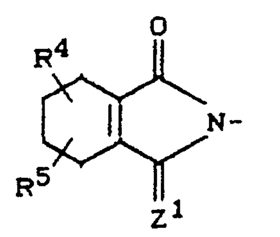 ; 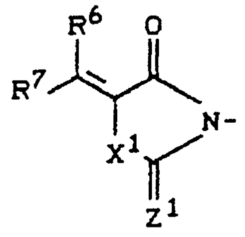 ; 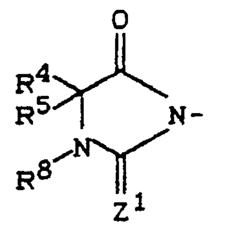 ;

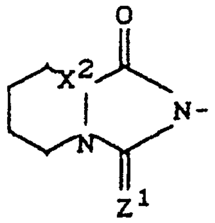 ; 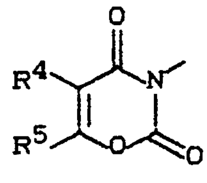 ; 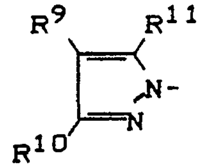 ;

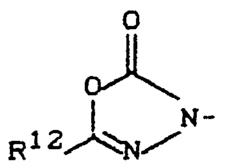 ; oder 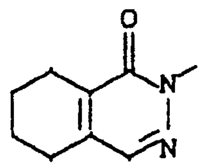

steht,

$R^1$ für Wasserstoff, Fluor, Chlor oder Brom steht,

$R^2$ für Wasserstoff, Methyl oder Ethyl steht,

$R^3$ für Methyl, Ethyl, n- oder i-Propyl, n-, i-, s-oder t-Butyl, für Allyl, Propargyl, für jeweils geradkettiges oder verzweigtes Pentyl, Hexyl, Butenyl, Pentenyl, Hexenyl, Butinyl, Pentinyl oder Hexinyl steht, außerdem für Halogenalkyl mit 1 bis 4 Kohlenstoffatomen oder Halogenalkenyl mit 3 bis 5 Kohlenstoffatomen und jeweils 1 bis 8 gleichen oder verschiedenen Halogenatomen, insbesondere Fluor, Chlor oder Brom steht, für Cyanalkyl, Alkoxyalkyl, Alkylthioalkyl, Halogenalkoxyalkyl, Alkoxyalkoxyalkyl, Alkylsulfonyl, Alkylcarbonyl, Alkoxycarbonyl, Alkylcarbonylalkyl, Alkoxycarbonylalkyl oder Alkoxyalkoxycarbonylalkyl mit jeweils 1 bis 5 Kohlenstoffatomen in den einzelnen Alkylteilen steht, außerdem für jeweils gegebenenfalls ein- bis dreifach, gleich und verschieden durch Methyl, Methoxy, Fluor oder Chlor substituiertes Cyclopropylmethyl, Cyclopentylmethyl, Cyclohexylmethyl, Cyclopropyloxycarbonylmethyl, Cyclopentyloxycarbonylmethyl, Cyclohexyloxycarbonylmethyl, Cyclopropyl, Cyclopentyl oder Cyclohexyl steht, für jeweils gegebenenfalls durch Methyl und/oder Ethyl substituiertes Oxetanylmethyl, Oxetanylethyl, Tetrahydrofuranylmethyl, Tetrahydrofuranylethyl, Tetrahydropyranylmethyl oder Tetrahydropyranylethyl steht oder für jeweils gegebenenfalls ein- bis dreifach, gleich oder verschieden substituiertes Benzyl oder Phenylethyl steht, wobei als Substituenten jeweils in Frage kommen: Fluor, Chlor, Brom, Cyano, Nitro, Methyl, Ethyl, n-oder i-Propyl, n-, i-, s- oder t-Butyl, Methoxy, Ethoxy, n- oder i-Propoxy, Methylthio, Ethylthio, Methoxycarbonyl, Ethoxycarbonyl, Trifluormethyl, Trifluormethoxy oder Trifluormethylthio,

$R^4$ und $R^5$ unabhängig voneinander jeweils für Wasserstoff, Methyl, Ethyl, n- oder i-Propyl stehen,

$R^6$ und $R^7$ entweder unabhängig voneinander jeweils für Wasserstoff, Methyl, Ethyl, n- oder i-Propyl stehen oder gemeinsam für einen zweifach verknüpften Alkandiylrest mit 2 bis 5 Kohlenstoffatomen stehen,

$R^8$ für Wasserstoff, Methyl, Ethyl, n- oder i-Propyl oder für gegebenenfalls ein- bis dreifach, gleich oder verschieden substituiertes Phenyl steht, wobei als Substituenten in Frage kommen: Fluor, Chlor, Brom, Cyano, Nitro, Methyl, Ethyl, n- oder i-Propyl, n-, i-, s-oder t-Butyl, Methoxy, Ethoxy, n- oder i-Propoxy, Methylthio, Trifluormethyl, Trifluormethoxy oder Trifluormethylthio und

$R^9$ für Wasserstoff, Fluor, Chlor, Brom, Methyl, Ethyl, n- oder i-Propyl, n-, i-, s-oder t-Butyl, Fluormethyl, Difluormethyl, Trifluormethyl, Chlormethyl, Dichlormethyl, Trichlormethyl, Difluorchlormethyl oder Dichlorfluormethyl steht oder

$R^{10}$ für Wasserstoff, Methyl, Ethyl, n- oder i-Propyl, n-, i-, s- oder t-Butyl, Fluormethyl, Difluormethyl, Trifluormethyl, Chlormethyl, Dichlormethyl, Trichlormethyl, Difluorchlormethyl oder Dichlorfluormethyl steht

und

$R^9$ und $R^{10}$ gemeinsam für einen 1,3-Propandiylrest einen 1,4-Butandiylrest oder einen 1,5-Pentandiylrest stehen,

$R^{11}$ für Wasserstoff, Fluor, Chlor, Brom, Methyl, Ethyl, n- oder i-Propyl, n-, i-, s-oder t-Butyl, Fluormethyl, Difluormethyl, Trifluormethyl, Chlormethyl, Dichlormethyl, Trichlormethyl, Difluorchlormethyl oder Dichlorfluormethyl steht,

$R^{12}$ für Wasserstoff, Methyl, Ethyl, n- oder i-Propyl, n-, i-, s- oder t-Butyl, jeweils einfach, zweifach oder dreifach durch Fluor und/oder Chlor substituiertes Methyl, Ethyl oder t-Butyl, für Allyl, n- oder i-Butenyl, Chlorallyl, Dichlorallyl, Propargyl, Chlorpropargyl, Methoxymethyl oder jeweils gegebenenfalls ein-bis fünffach, gleich oder verschieden durch Fluor, Chlor, Methyl und/oder Methoxy substituiertes Cyclopropyl, Cyclopentyl oder Cyclohexyl steht,

$X^1$ für Sauerstoff, eine $CH_2$-Gruppe oder eine

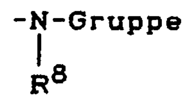

steht,

$X^2$ für Stickstoff oder eine CH-Gruppe steht, und

$Z^1$ für Sauerstoff oder Schwefel steht.

4. N-Aryl-Stickstoffheterocyclen der Formel (I) gemäß Anspruch 1, in welcher Het für

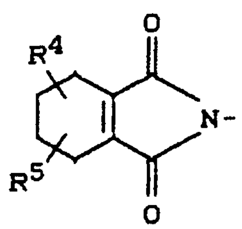

oder

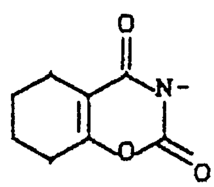

steht.

5. Verfahren zur Herstellung von N-Aryl-Stickstoffheterocyclen der Formel (I)

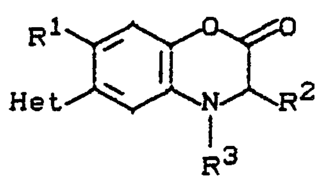

(I)

in welcher

Het für einen Heterocyclus der Formel

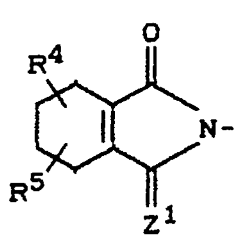 ; 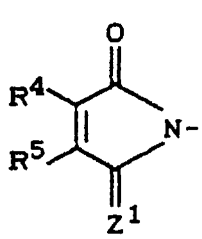 ; 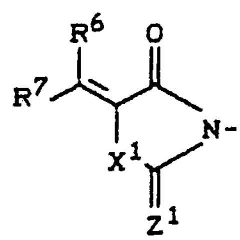 ;

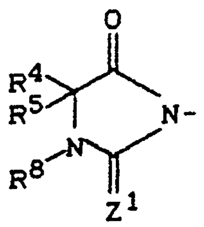 ; 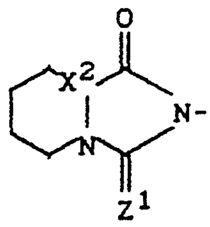 ; 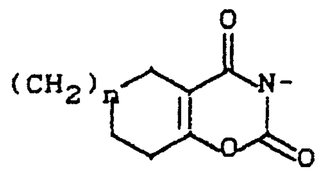 ;

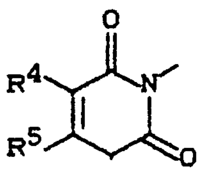 ; 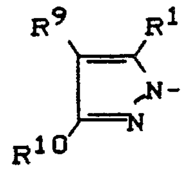 ; 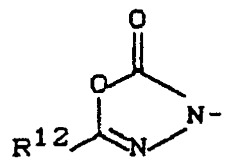 ;

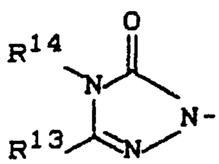 oder 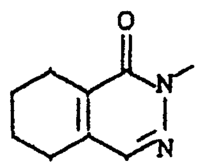 steht,

$R^1$ für Wasserstoff oder Halogen steht,
$R^2$ für Wasserstoff oder Alkyl steht,
$R^3$ für Wasserstoff oder jeweils gegebenenfalls substituiertes Alkyl, Alkenyl, Alkinyl, Cycloalkyl, Cycloalkenyl, Alkylsulfonyl, Arylsulfonyl, Alkylcarbonyl oder Alkoxycarbonyl steht,
$R^4$ und $R^5$ unabhängig voneinander jeweils für Wasserstoff oder Alkyl stehen,
$R^6$ und $R^7$ entweder unabhängig voneinander jeweils für Wasserstoff oder Alkyl stehen oder gemeinsam für einen zweifach verknüpften Alkandiylrest stehen,
$R^8$ für Wasserstoff, Alkyl oder gegebenenfalls substituiertes Aryl steht,
$R^9$ für Wasserstoff, Halogen, Alkyl oder Halogenalkyl steht,
$R^{10}$ für Wasserstoff, Alkyl oder Halogenalkyl steht oder
$R^9$ und $R^{10}$ gemeinsam für zweifach verknüpftes Alkandiyl stehen,
$R^{11}$ für Wasserstoff, Halogen, Alkyl oder Halogenalkyl steht,
$R^{12}$ und $R^{13}$ unabhängig voneinander für Wasserstoff, Alkyl, Alkoxyalkyl, Halogenalkyl, Alkenyl, Halogenalkenyl, Alkinyl, Halogenalkinyl oder für gegebenenfalls substituiertes Cycloalkyl steht,
$R^{14}$ für Wasserstoff, Alkyl, Halogenalkyl, Alkenyl, Halogenalkenyl, Alkinyl oder Halogenalkinyl steht oder
$R^{13}$ und $R^{14}$ gemeinsam für zweifach verknüpftes Alkandiyl stehen,
$X^1$ für Sauerstoff, eine $-CH_2$-Gruppe oder eine

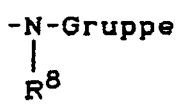

steht,
$X^2$ für Stickstoff oder eine CH-Gruppe steht,
$Z^1$ für Sauerstoff oder Schwefel steht und
n für die Zahl 0 oder 1 steht,
dadurch gekennzeichnet, daß man
(a) N-Aryl-Stickstoffheterocyclen der Formel (Ia)

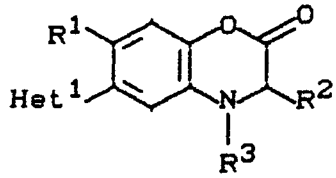

(Ia)

in welcher
Het[1] für einen Heterocyclus der Formel

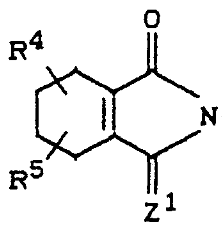

;

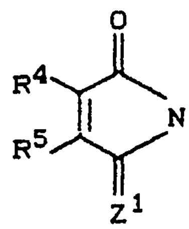

;

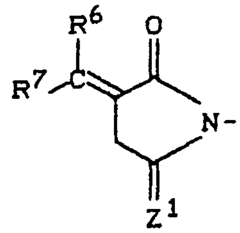

steht,
erhält, wenn man Anhydride der Formel (II),

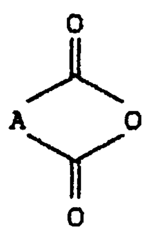

(II)

in welcher
A für einen Rest der Formel

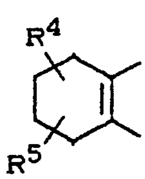

;

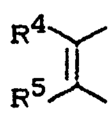

oder

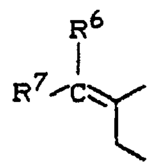

steht,

mit Aminobenzoxazinonen der Formel (III),

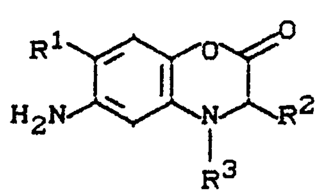

(III)

gegebenenfalls in Gegenwart eines Verdünnungsmittels und gegebenenfalls in Gegenwart eines Reaktionshilfsmittels umsetzt; oder daß man

(b) N-Aryl-Stickstoffheterocyclen der Formel (Ib),

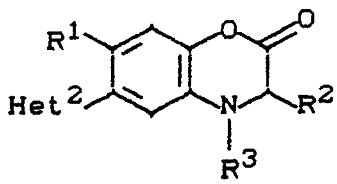

(Ib)

in welcher
$Het^2$ für einen Heterocyclus der Formel

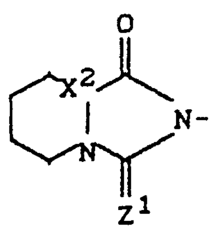

;

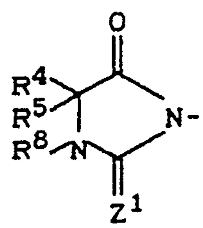

;

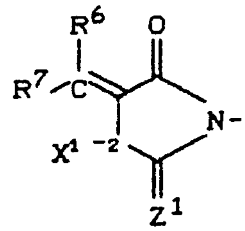

steht, wobei
$X^{1-2}$ für Sauerstoff oder eine

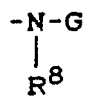-Gruppe

steht,
erhält, wenn man Carbonsäureester der Formel (IV),
$R^{15}$-$COOR^{16}$ (IV)
in welcher
$R^{15}$ für einen Rest der Formel

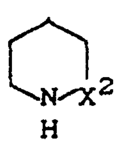

;

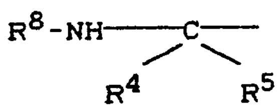

oder

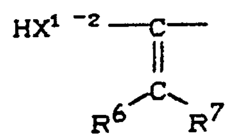

steht und
$R^{16}$ für Alkyl steht,
oder deren Säureadditionssalze
mit Iso(thio)cyanaten der Formel (V),

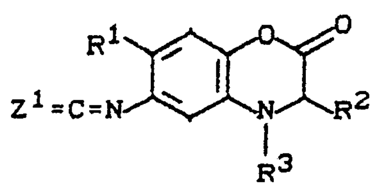

(V)

gegebenenfalls in Gegenwart eines Verdünnungsmittels und gegebenenfalls in Gegenwart eines Reaktionshilfsmittel, umsetzt; oder daß man
(c) N-Aryl-Stickstoffheterocyclen der Formel (Ic),

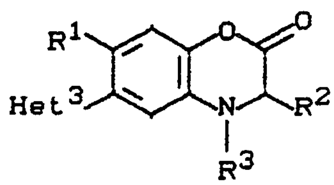

(Ic)

in welcher
$Het^3$ für einen Heterocyclus der Formel

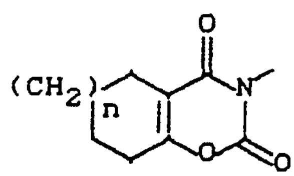

;

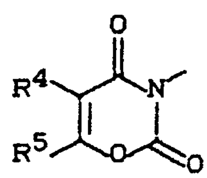

steht,
erhält, wenn man Verbindungen der Formeln (VIa) oder (VIb)

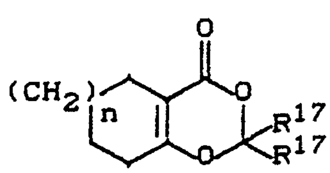

;

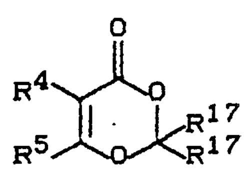

(VIa) (VIb)

in welcher
$R^{17}$ für Alkyl, vorzugsweise für $C_{1-4}$-Alkyl, insbesondere Methyl oder Ethyl steht,
mit Isocyanaten der Formel (Va)

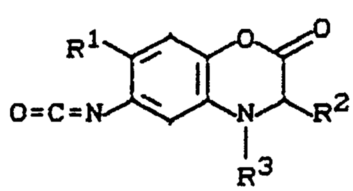

(Va)

in welcher

$R^3$ mit Ausnahme von Wasserstoff die oben angegebene Bedeutung hat, gegebenenfalls in Gegenwart eines Verdünnungsmittels und gegebenenfalls in Gegenwart eines Reaktionshilfsmittels umsetzt; oder daß man
(d) N-Aryl-Stickstoffheterocyclen der Formel (Id)

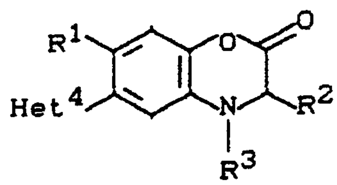

(Id)

in welcher
$Het^4$ für einen Heterocyclus der Formel

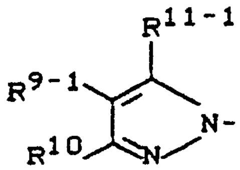

steht, wobei
$R^{9-1}$ für Wasserstoff, Alkyl oder Halogenalkyl oder gemeinsam mit $R^{10}$ für einen zweifach verknüpften Alkandiylrest steht und
$R^{11-1}$ für Wasserstoff, Alkyl oder Halogenalkyl steht und
$R^{10}$ die oben angegebene Bedeutung hat,
erhält, wenn man Phenylhydrazine der Formel (VII)

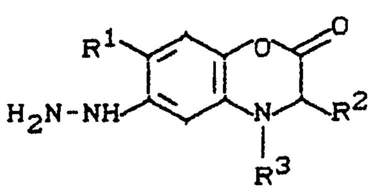

(VII)

mit 1,3-Diketonen der Formel (VIII),

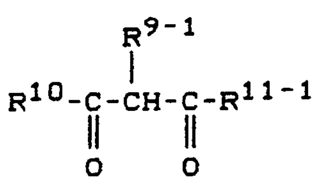

oder mit Derivaten dieser Diketone, wie Enolethern, Enolestern, Ketalen, Enoletherketalen, Enaminen oder β-Halogenvinylketonen gegebenenfalls in Gegenwart eines Verdünnungsmittels und gegebenenfalls in Gegenwart eines Reaktionshilfsmittels umsetzt; oder daß man
(e) N-Aryl-Stickstoffheterocyclen der Formel (Ie)

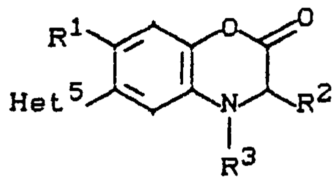

(Ie)

in welcher
Het[5] für einen Heterocyclus der Formel

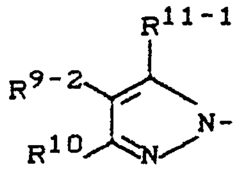

steht und
$R^{9-2}$ für Halogen steht,
erhält, wenn man N-Aryl-Stickstoffheterocyclen der Formel (If)

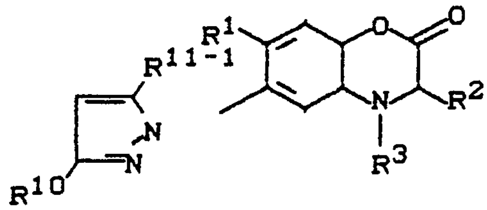

(If)

mit einem Halogenierungsmittel gegebenenfalls in Gegenwart eines Verdünnungsmittels umsetzt; oder daß man
(f) N-Aryl-Stickstoffheterocyclen der Formel (Ig)

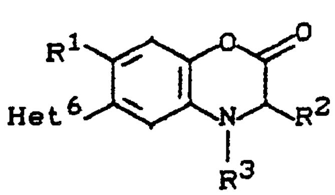

(Ig)

in welcher
Het[6] für einen Heterocyclus der Formel

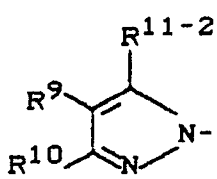

steht, wobei

$R^{11-2}$ für Halogen steht,
erhält, wenn man N-Aryl-pyrazolinone der Formel (IX),

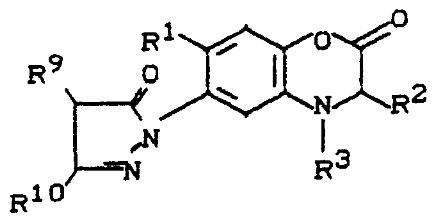

(IX)

mit einem Halogenierungsmittel, gegebenenfalls in Gegenwart eines Verdünnungsmittels und ge gebenenfalls in Gegenwart eines Reaktionshilfsmittels umsetzt; oder daß man
(g) N-Aryl-Stickstoffheterocyclen der Formel (Ih)

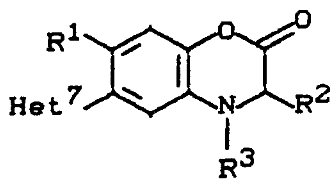

(Ih)

in welcher
$Het^7$ für einen Heterocyclus der Formel

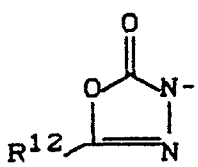

steht,
erhält, wenn man Phenylhydrazide der Formel (X),

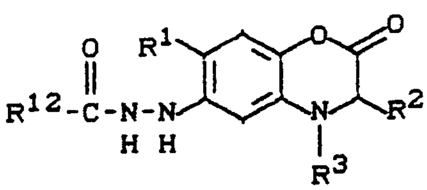

(X)

mit Phosgen gegebenenfalls in Gegenwart eines Verdünnungsmittels und gegebenenfalls in Gegenwart eines Reaktionshilfsmittels umsetzt; oder daß man
(h) N-Aryl-Stickstoffheterocyclen der Formel (Ii)

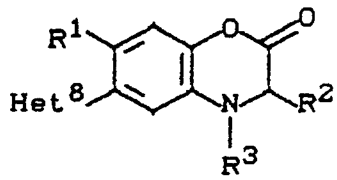

(Ii)

in welcher
$Het^8$ für einen Heterocyclus der Formel

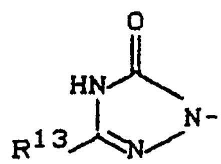

steht,
erhält, wenn man Phenylhydrazine der Formel (VII)

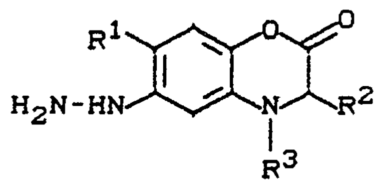

(VII)

mit Iminocarbonestern der Formel (XI)

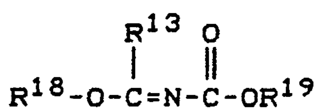

(XI)

in welcher
$R^{18}$ und $R^{19}$ unabhängig voneinander jeweils für Alkyl stehen,
gegebenenfalls in Gegenwart eines Verdünnungsmittels umsetzt; oder das man
(i) N-Aryl-Stickstoffheterocyclen der Formel (Ij)

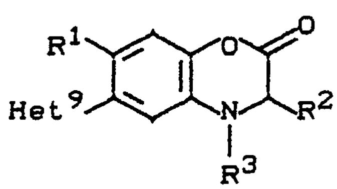

(Ij)

in welcher
$Het^9$ für einen Heterocyclus der Formel

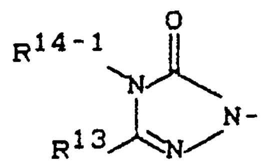

steht, in welcher
$R^{14-1}$ für Alkyl, Halogenalkyl, Alkenyl, Halogenalkenyl, Alkinyl oder Halogenalkinyl steht, erhält, wenn man N-Aryl-Stickstoffheterocyclen der Formel (Ii)

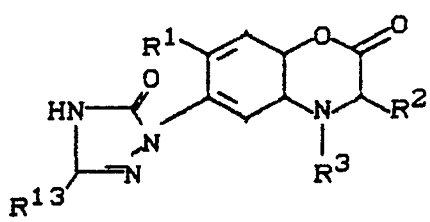

(Ii)

mit Alkylierungsmitteln der Formel (XII),
$R^{14-1}$-$E^1$ (XII)
in welcher
$R^{14-1}$ die oben angegebene Bedeutung hat und
$E^1$ für einen elektronenanziehende Abgangsgruppe steht,
gegebenenfalls in Gegenwart eines Verdünnungsmittels und gegebenenfalls in Gegenwart eines Reaktionshilfsmittels umsetzt; oder daß man
(j) N-Aryl-Stickstoffheterocyclen der Formel (Ik)

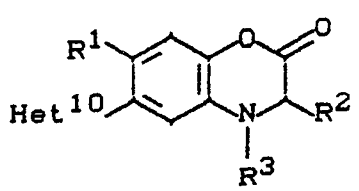

(Ik)

in welcher
$Het^{10}$ für einen Heterocyclus der Formel

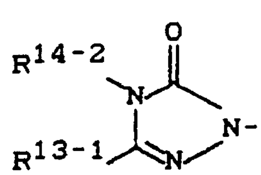

steht, wobei
$R^{13-1}$ und $R^{14-2}$ gemeinsam für einen zweifach verknüpften Alkandiylrest stehen, erhält, wenn man Amidrazone der Formel (XIII)

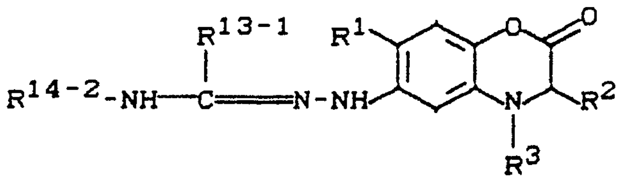

(XIII)

mit Phosgen gegebenenfalls in Gegenwart eines Verdünnungsmittels und gegebenenfalls in Gegenwart eines Reaktionshilfsmittels umsetzt; oder daß man
(k) N-Aryl-Stickstoffheterocyclen der Formel (II)

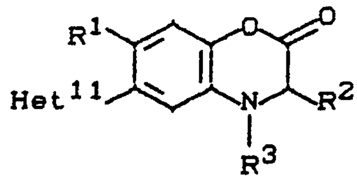

(I1)

in welcher
$Het^{11}$ für einen Heterocyclus der Formel

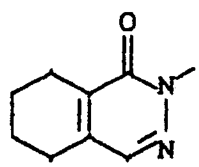

steht,
erhält, wenn man Hydroxyisobenzofuranon der Formel (XIV)

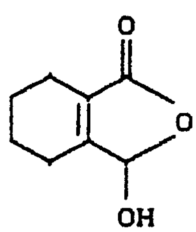

(XIV)

mit Phenylhydrazinen der Formel (VII)

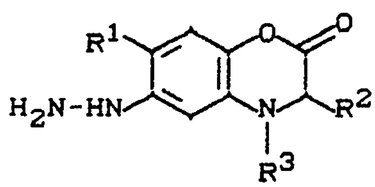

(VII)

gegebenenfalls in Gegenwart eines Verdünnungsmittels und gegebenenfalls in Gegenwart eines Reaktionshilfsmittels umsetzt; oder daß man
(l) N-Aryl-Stickstoffheterocyclen der Formel (Im)

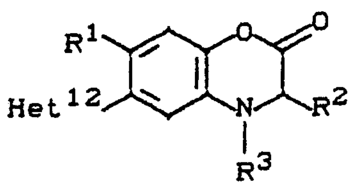

(Im)

in welcher
$Het^{12}$ für einen Heterocyclus der Formel

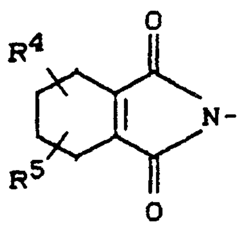 ; 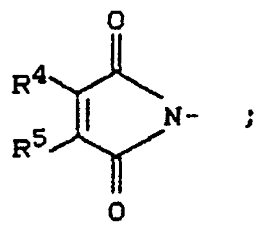 ; 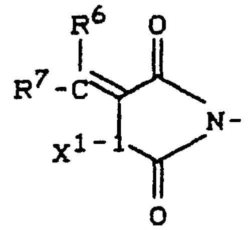 ;

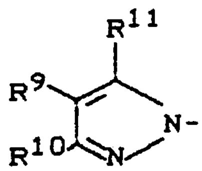

steht,
erhält, wenn man N-Aryl-Stickstoffheterocyclen der Formel (In)

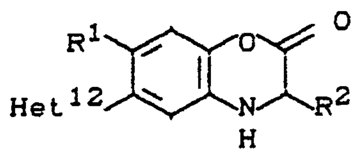

(In)

mit Alkylierungs- bzw. Acyclierungsmitteln der Formel (XVI)
$R^3$-$E^2$ (XVI)
in welcher
$R^3$ mit Ausnahme von Wasserstoff die oben angegebene Bedeutung hat und
$E^2$ für einen elektronenanziehende Abgangsgruppe steht,
gegebenenfalls in Gegenwart eines Verdünnungsmittels und gegebenenfalls in Gegenwart eines Reaktionshilfsmittels umsetzt.

6. Herbizide Mittel, gekennzeichnet durch einen Gehalt an mindestens einem N-Aryl-Stickstoffheterocyclus der Formel (I) gemäß Anspruch 1 oder 5.

7. Verfahren zur Bekämpfung von unerwünschten Pflanzen, dadurch gekennzeichnet, daß man H-Aryl-Stickstoffheterocyclen der Formel (I) gemäß Anspruch 1 oder 5 auf die Pflanzen und/oder ihren Lebensraum einwirken läßt.

8. Verwendung von N-Aryl-Stickstoffheterocyclen der Formel (I) gemäß Anspruch 1 oder 5 zur Bekämpfung von unerwünschten Pflanzen.

9. Verfahren zur Herstellung von herbiziden Mitteln, dadurch gekennzeichnet, daß man N-Aryl-Stickstoffheterocyclen der Formel (I) gemäß Anspruch 1 oder 5 mit Streckmitteln und/oder oberflächenaktiven Substanzen vermischt.

10. Verbindungen der Formel (IIIa)

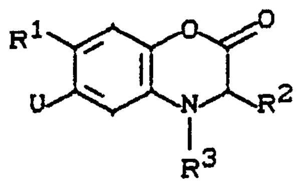

(IIIa)

in welcher
U für $-NH_2$ oder $-NH-NH_2$ steht,
$R^1$ für Wasserstoff oder Halogen steht,
$R^2$ für Wasserstoff oder Alkyl steht und
$R^3$ für Wasserstoff oder jeweils gegebenenfalls substituiertes Alkyl, Alkenyl, Alkinyl, Cycloalkyl, Cycloalkenyl, Alkylsulfonyl, Arylsulfonyl, Alkylcarbonyl oder Alkoxycarbonyl steht.

11. N-Aryl-pyrazolinone der Formel (IX)

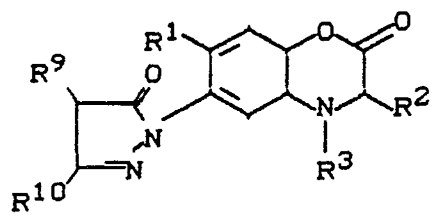

(IX)

in welcher
$R^1$ für Wasserstoff oder Halogen steht,
$R^2$ für Wasserstoff oder Alkyl steht,
$R^3$ für Wasserstoff oder jeweils gegebenenfalls substituiertes Alkyl, Alkenyl, Alkinyl, Cycloalkyl, Cycloalkenyl, Alkylsulfonyl, Arylsulfonyl, Alkylcarbonyl oder Alkoxycarbonyl steht,
$R^9$ für Wasserstoff, Halogen, Alkyl oder Halogenalkyl steht und
$R^{10}$ für Wasserstoff, Alkyl oder Halogenalkyl steht.

12. Phenylhydrazide der Formel (X)

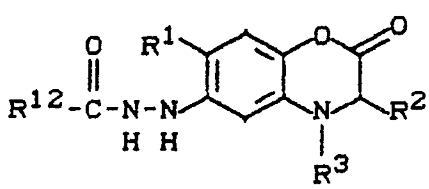

(X)

in welcher
$R^1$ für Wasserstoff oder Halogen steht,
$R^2$ für Wasserstoff oder Alkyl steht,
$R^3$ für Wasserstoff oder jeweils gegebenenfalls substituiertes Alkyl, Alkenyl, Alkinyl, Cycloalkyl, Cycloalkenyl, Alkylsulfonyl, Arylsulfonyl, Alkylcarbonyl oder Alkoxycarbonyl steht und
$R^{12}$ für Wasserstoff, Alkyl, Alkoxyalkyl, Halogenalkyl, Alkenyl, Halogenalkenyl, Alkinyl, Halogenalkinyl oder für gegebenenfalls substituiertes Cycloalkyl steht.

13. Amidrazone der Formel (XIII)

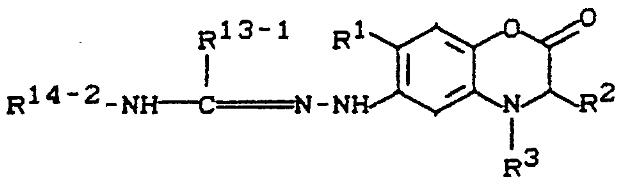

(XIII)

in welcher
$R^1$ für Wasserstoff oder Halogen steht,
$R^2$ für Wasserstoff oder Alkyl steht,
$R^3$ für Wasserstoff oder jeweils gegebenenfalls substituiertes Alkyl, Alkenyl, Alkinyl, Cycloalkyl, Cycloalkenyl, Alkylsulfonyl, Arylsulfonyl, Alkylcarbonyl oder Alkoxycarbonyl steht und
$R^{13-1}$ und $R^{14-2}$ gemeinsam für einen zweifach verknüpften Alkandiylrest stehen.